# EUROPEAN PATENT SPECIFICATION

(11) **EP 3 673 911 B1**
(45) Date of publication and mention of the grant of the patent: **20.03.2024**
(21) Application number: 20154049.9
(22) Date of filing: 18.08.2017
(51) Int. Cl.: A61K 35/17, C12N 5/0783, A61K 31/495, A61K 39/395

(54) **COMPOSITIONS AND METHODS FOR CANCER IMMUNOTHERAPY**
ZUSAMMENSETZUNGEN UND VERFAHREN FÜR DIE KREBSIMMUNTHERAPIE
COMPOSITIONS ET MÉTHODE POUR L'IMMUNOTHÉRAPIE DU CANCER

(30) Priority: 18.08.2016 US 201662376680 P
(43) Date of publication of application: 01.07.2020
(62) Divisional of application: 17842178.0
(73) Proprietor: The UAB Research Foundation, Birmingham, AL 35233 (US); IN8bio, Inc., New York, NY 10118 (US)
(72) Inventor: HO, William, New York, NY 10001 (US); LAMB, Lawrence S., Birmingham, AL 35242 (US)
(74) Representative: Lee, Nicholas John

(56) References cited:
- LAMB L.S. ET AL: "Engineered Drug Resistant [gamma][delta] T Cells Kill Glioblastoma Cell Lines during a Chemotherapy Challenge: A Strategy for Combining Chemo- and Immunotherapy", PLOS ONE, vol. 8, no. 1, 11 January 2013 (2013-01-11), page e51805, XP055127744,
- KAMATA T. ET AL.: "Blockade of programmed death-1/programmed death ligand pathway enhances the antitumor immunity of human invariant natural killer T cells", CANCER IMMUNOL. IMMUNOTHER., vol. 65, 15 September 2016 (2016-09-15), pages 1477-1489, XP002797577,
- DONDERO A. ET AL: "PD-L1 expression in metastatic neuroblastoma as an additional mechanism for limiting immune surveillance", ONCOIMMUNOLOGY, vol. 5, no. 1, 2 January 2016 (2016-01-02) , page e1064578, XP055667574,
- ANTONIOS J.P. ET AL: "Immunosuppressive tumor-infiltrating myeloid cells mediate adaptive immune resistance via a PD-1/PD-L1 mechanism in glioblastoma", NEURO-ONCOLOGY, vol. 19, no. 6, 23 January 2017 (2017-01-23), pages 796-807, XP055667585,
- PILLAI S.: "Engineered-drug resistant gamma-delta T cells combined with immune checkpoint blockade augmented killing of cancer cells.", NEURO-ONCOLOGY, vol. 20, no. S6, IMMU-15, November 2018 (2018-11), page vi124,
- PILLAI S.: "Chemotherapy, checkpoint inhibition, and MGMT-modified adoptive gamma-delta T cell-based therapy to treat post-resection, primary glioblastomas.", CANCER RES., vol. 79, no. 13S, 2262, July 2019 (2019-07),

## Description

### BACKGROUND OF THE INVENTION

Although outstanding progress has been made in the fields of cancer detection and therapy, the treatment of late-stage and metastatic cancer remains a major challenge. Cytotoxic chemotherapy agents remain among the most used and successfully employed anti-cancer treatments. However, they are not uniformly effective, and the introduction of these agents with novel therapies, such as immunotherapies, is problematic. For example, chemotherapy agents can be detrimental to the establishment of robust anti-tumor immunocompetent cells due to the agents' non-specific toxicity profiles. Small molecule-based therapies targeting cell proliferation pathways may also hamper the establishment of anti-tumor immunity. Further complications found in immunotherapeutic approaches is the ability of tumor cells to outsmart the body's immune response through the downregulation of MHC-class I antigen expression, presentation of immune checkpoint molecules on the tumor or effector cell surfaces or the shedding of soluble tumor ligands into the plasma leading to effector cell receptor downregulation. Such processes lead to a tumor that appears normal resulting in the evasion of an immune system attack. However, if chemotherapy regimens that are transiently effective can be combined with both novel immunocompetent cell therapies and immune checkpoint inhibitors, then significant improvement in anti-neoplastic therapy might be achieved.

Several drug resistant genes have been identified that can potentially be used to confer drug resistance to targeted immune cells, and advances in gene therapy techniques have made it possible to test the feasibility of using these genes in drug resistance gene therapy studies. For example, an shRNA strategy was used to decrease the levels of hypoxanthine-guanine phosphoribosyltransferase (HPRT), which conferred resistance to 6-thioquanine. Also, the drug resistant gene MGMT encoding human alkyl guanine transferase (hAGT) is a DNA repair protein that confers resistance to the cytotoxic effects of alkylating agents, such as nitrosoureas and temozolomide (TMZ). 6-benzylguanine (6-BG) is an inhibitor of AGT that potentiates nitrosourea toxicity and is co-administered with TMZ to potentiate the cytotoxic effects of this agent. Several mutant forms of MGMT that encode variants of AGT are highly resistant to inactivation by 6-BG, but retain their ability to repair DNA. P140KMGMT-based drug resistant gene therapy has been shown to confer chemoprotection to mouse, canine, rhesus macaques, and human cells, specifically hematopoietic cells.

Tumor cells often express cell-surface molecules that reveal their cancerous nature. However, these same cells may also present immune checkpoint molecules on their surfaces that mimic those of normal cells, thereby avoiding an immune attack. Immune checkpoints are often regulated by interactions between specific receptor/ligand pairs including CTLA-4 and PD-1. CTLA-4, PD-1 and its ligands are members of the CD28-B7 family of cosignaling molecules that play important roles throughout all stages of T-cell function and other cell functions. The PD-1 receptor is expressed on the surface of activated T cells (and B cells) and, under normal circumstances, binds to its ligands (PD-L1 and PD-L2) that are expressed on the surface of antigen-presenting cells, such as dendritic cells or macrophages. This interaction sends a signal into the T cell and essentially switches it off or inhibits it. Cancer cells take advantage of this system by driving high levels of expression of PD-L1 on their surface. This allows them to gain control of the PD-1 pathway and switch off T cells expressing PD-1 that may enter the tumor microenvironment, thus suppressing the anticancer immune response.

Checkpoint inhibitors provide a means to unmask tumor cells by blocking receptor/ligand pairs such as CTLA-4 or PD-1, allowing the T cells to mount an immune response. Six immune checkpoint inhibitors that have received accelerated approval from the U.S. Food and Drug Administration for cancer are ipilimumab (YERVOY^{®}), pembrolizumab (KEYTRUDA^{®}), atezolizumab (TECENTRIQ^{®}), durvalumab (IMFINZI^{™}), avelumab (BAVENCIO), and nivolumab (OPDIVO^{®}). YERVOY^{®} is a monoclonal antibody that targets CTLA-4 on the surface of T cells and is approved for the treatment of melanoma. KEYTRUDA^{®} targets PD-L1 and is used to treat melanoma and non-small cell lung cancer. OPDIVO^{®} also targets PD-1 and is approved for treatment of melanoma, renal cell carcinoma, and non-small cell lung cancer. Additional checkpoint targets that may prove to be effective are TIM-3, LAG-3, various B-7 ligands, CHK 1 and CHK2 kinases, BTLA, A2aR, and others.

Typically, activity and high objective response rates (ORR) in connection with checkpoint inhibitor treatment is associated with tumors with high mutational loads. Mutation frequency is generally correlated with high neoantigen signaling resulting in increased tumor immunogenicity.

In May 2017, The U.S. Food and Drug Administration (FDA) granted accelerated approval to pembrolizumab, for adult and pediatric patients with unresectable or metastatic, microsatellite instability-high (MSI-H) or mismatch repair deficient (dMMR) solid tumors that have progressed following prior treatment and who have no satisfactory alternative treatment options or with MSI-H or dMMR colorectal cancer that has progressed following treatment with a fluoropyrimidine, oxaliplatin, and irinotecan. This is the FDA's first approval for a tissue/site agnostic indication. Patients with dMMR have a defect that confers an inability to correct genetic mutations that leads to a hypermutated state.

Clinical data has demonstrated that in cancers such as colorectal cancer where the ORR is typically 0% with checkpoint inhibitor treatment, ORR's are increased to 62% in those with dMMR due to their high mutational burden (Le DT, et al. ASCO 2015. Abstract LBA100). Similarly, an analysis by Hodges et al. (Neuro-Oncology, 19(8), 1047-1057, 2017) demonstrates that glioblastomas (GBMs) typically have a low mutation burden with only 3.5% of those GBMs analyzed demonstrating a high tumor mutational load. In those GBM patients with dMMR, however, they have been found to have the highest mutation load over other high-grade tumors by Boufett et al. (JCO, 2016 34:19, 2206-2211). Boufett et al. describe the remarkable and durable responses of two pediatric patients with recurrent multifocal GBM who were refractory to current standard therapies when treated with single-agent nivolumab. A dMMR mutation drove these two patients to responses with checkpoint inhibition, but their failed responses to standard therapies may have also been due to dMMR. This supports data suggesting that generally GBM's are not significantly immunogenic and are not likely to demonstrate significant ORR's when treated with checkpoint inhibitors.

Since 2005, standard-of-care treatment for front-line GBM has been the Stupp Protocol, involving treatment with the chemotherapy temozolomide (TMZ) (Stupp et al., N Engl J Med 2005; 352:987-996 March 10, 2005). TMZ is an alkylating agent that causes double stranded DNA breaks. TMZ functions by creating O⁶-methylguanine (O⁶MeG) adducts in DNA which will cause a mispairing and formation of a GC to AT point mutation (Roos et al., Cancer Letters 332 (2013) 237). The resistance mechanism to effective treatment with TMZ is O⁶-methylguanine-methyltransferase (MGMT), which removes the methyl adduct from the O⁶ position. In cells that have a functioning MMR system, the GC to AT point mutation will cause a double stranded break after two cycles of DNA replication. As Roos notes, "O⁶MeG does not trigger apoptosis directly; it requires MMR and DNA replication." Thus, those newly diagnosed GBM patients who are most likely to respond to immunotherapy and/or checkpoint inhibition due to high mutational load and neoantigen burden, are also those least likely to respond to conventional alkylating chemotherapies. This realization would explain the observation that MMR and MLH1 mutations are actually a negative prognosticator for survival in GBM patients (Draaisma et al., Acta Neuropathol Commun. 2015; 3: 88). These patients don't respond to conventional therapy with TMZ.

The present invention provides a solution to this challenge. While hypermutations due to dMMR can lead to stronger immunogeneic signaling, they also likely result in low responses to standard chemotherapeutic agents and low survival. By causing double stranded-DNA breaks, TMZ has demonstrated the ability to drive hypermutations in tumors and stronger immunogeneic signaling. By artificially creating hypermutations with chemotherapy, one can maintain tumor sensitivity to standard treatment while increasing tumor immunogenicity and responsiveness to checkpoint inhibition if one can also keep the lymphocytes alive during chemotherapy treatment.

Unfortunately, over time TMZ driven hypermutations are likely to cause mutations in the MMR system, leading to decreased sensitivity to TMZ and other alkylating agents. Without being bound by theory, the hypermutations caused by TMZ or other alkylating agents are most likely to be subclonal signature 11 mutations that may not elicit long term immunogenicity (Alexandrov et al., Nature, Vol 500, 22 Aug. 2013). Shorter term however, our data demonstrates that the initiation of the double stranded break can trigger a DNA damage response that results in the transient upregulation of the NKG2D receptor ligands on the tumor cell surface and trigger activation of an innate immune response and tumor killing by drug resistant γδ T-cells (Lamb et al., PLOS ONE, Vol 8, Issue 1, Jan. 2013).

The present invention thus targets both mechanisms together early on in the progression of cancer. Without being bound by theory, the DNA damage caused by TMZ results in the upregulation of *ataxia telangiectasia mutated* (ATM) and *ataxia telangiectasia Rad-3 related* (ATR) protein kinases associated to the DNA damage response. The DNA damage response and upregulation of ATM and ATR results in the upregulation of NKG2D ligands such as MICA/B and the ULBP16 on the tumor cell surface and the initiation of an anti-tumor immune response. By killing most of the tumor that is sensitive to chemotherapy while upregulating the immune response through DNA damage and taking the brakes off of the immune cells with checkpoint inhibition, we seek to create stronger, more durable responses for cancer patients.

Accordingly, there remains an urgent need for combination therapies that enhance, replace or supplement current methods of treating cancers, and in particular those cancers that exhibit transient responses to chemotherapy. Combination therapies comprising drug-resistant immunocompetent cells, immune checkpoint inhibitors, and chemotherapy offers such a supplemental approach.

Relevant prior art information can be found in:
Lamb (2013); PlosOne 8(1):e51805
Kamata (2016); Cancer Immunol. Immunother. 65:1477-1489
Dondero (2016); Oncoimmunology
Antonios (2017); Neuro-Oncology 19(6):796-807

### SUMMARY OF THE INVENTION

Aspects of the invention for which protection is sought are defined in the appended set of claims. The description may contain additional technical information, which although not part of the claimed invention, is provided to place the invention in a broader technical context and to illustrate possible related technical developments. References herein to methods of treatment of the human or animal body are to be understood as references to medicaments for use in a method of treatment.

The invention provides at least one immune checkpoint inhibitor for use in a method for the treatment of cancer in a patient in need thereof, the method comprising the steps of:
i. obtaining an expanded population of isolated cytotoxic immune cells comprising γδ T-cells, wherein greater than about 50% of the γδ T-cells have been genetically modified to express a polypeptide that confers resistance to a chemotherapeutic agent;
ii. administering to the patient an effective amount of the chemotherapeutic agent to which the genetically modified cytotoxic immune cells of step (i) are resistant;
iii. administering to the patient the population of genetically modified cytotoxic immune cells of step (i); and
iv. administering to the patient an effective amount of the at least one immune checkpoint inhibitor wherein the checkpoint inhibitor targets PD-1, PDL1, or CTLA-4.

The present invention provides combination therapies for treating cancer comprising compositions and methods for enhancing the immune response of immunocompetent cells against cancer, including protection from drug-induced toxicities during chemotherapy, thereby allowing for the combined administration of immuno- and chemotherapy, an anticancer treatment termed "drug resistant immunotherapy" in combination with one or more cycles and/or doses of an immune checkpoint inhibitor. The combination of checkpoint inhibitors and drug resistant immunotherapy may be administered sequentially in any order, or substantially simultaneously. Drug resistant-immunotherapy comprises genetic modification of isolated cytotoxic immune cells. Preferably, the isolated cytotoxic immune cells are genetically modified using any method known in the art. Preferably the method of genetic modification includes, but is not limited to, an HIV-based lentiviral system or a gene editing system. Preferably the gene editing system comprises the use of directed endonucleases, including, but not limited to, Zinc Finger Nucleases (ZFNs), Transcription Activator Like Effector Nucleases (TALENs), or proteins, like Cas9, associated with Clustered Regularly Interspaced Palindromic Repeats (CRISPR) to deliver the drug resistance-conferring genetic element into immunocompetent cell lines. Genetically engineered immunocompetent cells develop significant resistance to a specific chemotherapeutic cytotoxic agent compared to non-modified cells; however, the drug resistance does not affect the genetically engineered cell's ability to kill target cancer cells in the presence or absence of a chemotherapy agent. Such drug resistant immunotherapy is described, for example, by Spencer H.T. et al. in US 2015/0017137.

Disclosed herein are methods for treating cancer in a patient, comprising the steps of: obtaining an optionally enriched and/or optionally expanded population of cytotoxic immune cells, wherein the cytotoxic immune cells comprise cells that have been genetically modified to be resistant to a therapeutic agent; administering to a patient in need thereof, an effective amount of the therapeutic agent to which the genetically engineered cells are resistant in combination with the population of genetically modified cytotoxic immune cells, and further administering to the patient an effective amount of at least one immune checkpoint inhibitor, thereby treating cancer in the patient.

The population of cytotoxic immune cells used in the compositions and methods of the invention comprises γδ T-cells. Preferably the population of cytotoxic immune cells comprises about 10%, 20%, 30%, 40%, 50%, 60%, 70%, 75%, 80%, 85%, 90%, 95% or 100% γδ T-cells. Preferably the population of cytotoxic immune cells comprises about 50% to about 95% γδ T-cells. Preferably the population of cytotoxic immune cells used in the compositions and methods of the invention comprises γδ T-cells and natural killer (NK) cells. Preferably the population of cytotoxic immune cells comprises about 1%, 5%, 10%, 15%, 20%, 25%, 30%, 35%, 40%, 45%, 50%, or more of NK cells. Preferably the population of cytotoxic immune cells comprises about 5% to about 25% of NK cells. Preferably the population of cytotoxic immune cells used in the compositions and methods of the invention comprise γδ T-cells, NK cells and other immunocompetent cells including, but not limited to: monocytes, dendrites and macrophages. Preferably, the population of cytotoxic immune cells used in the compositions and methods of the invention comprise γδ T-cells derived from human induced pluripotent stem cells (hiPSCs).

Preferably, the step of obtaining a population of cytotoxic immune cells genetically modified to be resistant to a therapeutic agent comprises: obtaining from a subject such as a human subject or animal subject a population of cytotoxic immune cells, for example by obtaining a biological sample from the subject including but not limited to a blood or tissue sample including a tumor biopsy. The sample may optionally be enriched for cytotoxic immune cells and other immunocompetent cells and/or the cells present in the sample may optionally be expanded to increase the population of the cells present in the sample. The cytotoxic immune cells are preferably stably transformed or gene edited with a vector comprising a heterologous nucleic acid sequence operably linked to a promoter, wherein the heterologous nucleic acid sequence encodes a polypeptide conferring to the cell resistance to one or more chemotherapeutic agents.

Disclosed herein are systems for treating a cancer in a patient comprising a cytotoxic therapeutic agent having the characteristics of inhibiting the survival of a cancer cell, a population of cytotoxic immune cells comprising γδ T-cells, NK cells, or any combination thereof and optionally further comprising other immunocompetent cells and wherein the cytotoxic immune cells are genetically modified to be resistant to the cytotoxic therapeutic agent, and an immune checkpoint inhibitor which blocks cell-surface proteins on cancer cells.

Disclosed herein are systems for treating a glioblastoma in a patient comprising a therapeutic agent having the characteristics of inhibiting the survival of a cancer cell and inducing a stress protein in the cancer cell, a population of cytotoxic immune cells, wherein said cytotoxic immune cells comprise γδ T-cells, NK cells, or any combination thereof and optionally further comprise other immunocompetent cells and wherein said cytotoxic immune cells have been genetically modified to be resistant to the therapeutic agent, in combination with an immune checkpoint inhibitor which blocks cell-surface proteins on cancer cells.

The population of cytotoxic immune cells used in the invention comprise γδ T-cells wherein greater than about 50%, and preferably 60%, 70% 80%, 90%, or 95%, of the γδ T-cells express a polypeptide that confers resistance to a chemotherapy agent. Also disclosed herein are isolated compositions comprising γδ T-cells wherein greater than about 5%, 10%, 20%, 30%, 40%, 50%, 60%, 70% 80%, 90%, or 95% of the γδ T-cells comprise a nucleic acid that encodes a polypeptide that confers resistance to a chemotherapy agent, or isolated compositions consisting essentially οf γδ T-cells comprising a nucleic acid that encodes a polypeptide that confers resistance to a chemotherapy agent. Preferably, the polypeptide that confers resistance to a chemotherapy agent is O⁶ methylguanine DNA methyltransferase (MGMT), a drug resistant variant of dihydrofolate reductase (L22Y-DHFR), thymidylate synthase, and/or multiple drug resistance-1 protein (MDR1).

Disclosed herein are compositions comprising at least one checkpoint inhibitor, and an isolated population of cytotoxic immune cells comprising γδ T-cells NK cells, or any combination thereof, wherein greater than about 5%, and preferably greater than about 50% of the population of cytotoxic immune cells express a polypeptide that confers resistance to a therapeutic agent capable of inhibiting the survival of a cancer cell.

### BRIEF DESCRIPTION OF THE FIGURES

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 Shows checkpoint molecules expression on γδ T cells, before *in vitro* culture of PBMCs from Donor 1.
FIG. 2 Shows upregulation of checkpoint molecules on *in vitro* enriched γδ T cells from Donor 1.
FIG. 3 Shows checkpoint molecules expression on γδ T cells, before *in vitro* culture of PBMCs from Donor 2.
FIG. 4 Shows upregulation of checkpoint molecules on *in vitro* enriched γδ T cells from Donor 2.
FIG. 5 Shows upregulation of PD-1 and CTLA-4 on human γδ T cells upon stimulation with IL2 and Zol.
FIG. 6 Shows PD-L1 expression on glioblastoma tumor cells.
FIG. 7 Shows cytotoxicity of GMP manufactured γδ T cells.

### DETAILED DESCRIPTION

This invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit (unless the context clearly dictates otherwise), between the upper and lower limit of that range, and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

Unless otherwise indicated, the present specification describes techniques of chemistry, synthetic organic chemistry, biochemistry, biology, molecular biology, molecular imaging, and the like, which are within the skill of the art. Such techniques are explained fully in the literature.

The following examples are put forth so as to provide those of ordinary skill in the art with a complete invention and description of how to perform the methods and use the compositions and compounds disclosed and claimed herein.

Unless otherwise indicated, the present invention is not limited to particular materials, reagents, reaction materials, manufacturing processes, or the like, as such can vary. It is also to be understood that the terminology used herein is for purposes of describing particular features only, and is not intended to be limiting. It is also possible in the present invention that steps can be executed in different sequence where this is logically possible.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

### Definitions

In describing and claiming the disclosed subject matter, the following terminology will be used in accordance with the definitions set forth below.

By "administration" is meant introducing a compound, biological materials including a cell population, or a combination thereof, of the present invention into a human or animal subject. One preferred route of administration of the compounds is intravenous. Other preferred routes of administration of the compounds may be intraperitoneal or intrapleural, or via a catheter to the brain. However, any route of administration, such as oral, topical, subcutaneous, peritoneal, intra-arterial, inhalation, vaginal, rectal, nasal, introduction into the cerebrospinal fluid, or instillation into body compartments can be used. Direct injection into a target tissue site such as a solid tumor is also contemplated.

The term "therapeutic agent" as used herein refers to a compound or a derivative thereof that can interact with a cancer cell, thereby reducing the proliferative status of the cell and/or killing the cell. Examples of therapeutic agents include, but are not limited to, chemotherapeutic agents which include, but are not limited to, alkylating agents (e.g., cyclophosphamide, ifosfamide); metabolic antagonists (e.g., methotrexate (MTX), 5-fluorouracil or derivatives thereof); a substituted nucleotide; a substituted nucleoside; DNA demethylating agents (also known as antimetabolites; e.g., azacitidine); antitumor antibiotics (e.g., mitomycin, adriamycin); plant-derived antitumor agents (e.g., vincristine, vindesine, TAXOL^{®}, paclitaxel, abraxane); cisplatin; carboplatin; etoposide; and the like. Such agents may further include, but are not limited to, the anti-cancer agents trimethotrexate (TMTX); temozolomide; raltitrexed; S-(4-Nitrobenzyl)-6-thioinosine (NBMPR); 6-benzyguanidine (6-BG); nitrosoureas [e.g., bis-chloronitrosourea (BCNU; carmustine), lomustine (CCNU) +/- Procarbazine and Vincristine (PCV regimen), fotemustine]; cytarabine; and camptothecin; or a therapeutic derivative of any thereof.

The term "therapeutically effective amount" as used herein refers to that amount of the compound or therapeutically active composition being administered that will relieve to some extent one or more of the symptoms of a disease, a condition, or a disorder being treated. In reference to cancer or pathologies related to unregulated cell division, a therapeutically effective amount refers to that amount which has the effect of (1) reducing the size of a tumor, (2) inhibiting (that is, slowing to some extent, preferably stopping) aberrant cell division, for example cancer cell division, (3) preventing or reducing the metastasis of cancer cells, and/or, (4) relieving to some extent (or, preferably, eliminating) one or more symptoms associated with a pathology related to or caused in part by unregulated or aberrant cellular division, including for example, cancer, or angiogenesis.

The terms "treating" or "treatment" of a disease (or a condition or a disorder) as used herein refer to preventing the disease from occurring in a human subject or an animal subject that may be predisposed to the disease but does not yet experience or exhibit symptoms of the disease (prophylactic treatment), inhibiting the disease (slowing or arresting its development), providing relief from the symptoms or side-effects of the disease (including palliative treatment), and causing regression of the disease. With regard to cancer, these terms also mean that the life expectancy of an individual affected with a cancer may be increased or that one or more of the symptoms of the disease will be reduced. With regard to cancer, "treating" also includes enhancing or prolonging an anti-tumor response in a subject.

As used herein any form of administration of a "combination", "combined therapy" and/or "combined treatment regimen" refers to at least two therapeutically active drugs or compositions which may be administered simultaneously, in either separate or combined formulations, or sequentially at different times separated by minutes, hours or days, but in some way act together to provide the desired therapeutic response.

The term "enhancing", as used herein, refers to allowing a subject or tumor cell to improve its ability to respond to a treatment disclosed herein. For example, an enhanced response may comprise an increase in responsiveness of at least 5%, 10%, 15%, 20%, 25%, 30%, 35%, 40%, 45%, 50%, 55%, 60%, 65%, 70%, 75%, 80%, 85%, 90%, 95% or 98% or more. As used herein, "enhancing" can also refer to enhancing the number of subjects who respond to a treatment such as a combination therapy comprising chemotherapy, drug-resistant immunocompetent cells, and immune checkpoint inhibitors. For example, an enhanced response may refer to a total percentage of subjects who respond to a treatment wherein the percentage is of at least 5%, 10%, 15%, 20%, 25%, 30%, 35%, 40%, 45%, 50%, 55%, 60%, 65%, 70%, 75%, 80%, 85%, 90%, 95% or 98% or more.

The terms "subject" and "patient" as used herein include humans, mammals (e.g., cats, dogs, horses, etc.), living cells, and other living organisms. A living organism can be as simple as, for example, a single eukaryotic cell or as complex as a mammal. Typical patients are mammals, particularly primates, especially humans. For veterinary applications, a wide variety of subjects will be suitable, e.g., livestock such as cattle, sheep, goats, cows, swine, and the like; poultry such as chickens, ducks, geese, turkeys, and the like; and domesticated animals particularly pets such as dogs and cats. For diagnostic or research applications, a wide variety of mammals will be suitable subjects, including rodents (e.g., mice, rats, hamsters), rabbits, primates, and swine such as inbred pigs and the like. Preferably, a system includes a sample and a subject. The term "living host" refers to host or organisms noted above that are alive and are not dead. The term "living host" refers to the entire host or organism and not just a part excised (e.g., a liver or other organ) from the living host.

The term "yδ T-cells (gamma delta T-cells)" as used herein refers to a small subset of T-cells that express a distinct T-cell receptor (TCR) on their surface. A majority of T-cells have a TCR composed of two glycoprotein chains called α- and β-TCR chains. In contrast, in γδ T-cells, the TCR is made up of one γ-chain and one δ-chain. This group of T-cells is usually much less common than αβ T-cells, but are found at their highest abundance in the gut mucosa, within a population of lymphocytes known as intraepithelial lymphocytes (IELs). The antigenic molecules that activate γδ T-cells are still largely unknown. However, γδ T-cells are peculiar in that they do not seem to require antigen processing and MHC presentation of peptide epitopes although some recognize MHC class IB molecules. Furthermore, γδ T-cells are believed to have a prominent role in recognition of lipid antigens, and to respond to stress-related antigens such as, MIC-A and MIC-B.

The term "human induced pluripotent stem cells" (hiPSCs) as used herein refers to a type of pluripotent stem cells that can be generated directly from human adult cells such as skin or blood cells that have been reprogrammed back into an embryonic-like pluripotent state that enables the generation of any other type of human cell, for example a therapeutic immunocompetent cell. The human adult cells from which the hiPSCs may be obtained from the patient to be treated or the adult cells may be obtained from a different individual. Human adult cells may be transformed into pluripotent stem cells with, for example, a retroviral system or a lentiviral system for introducing genes encoding transcription factors that are able to convert adult cells into pluripotent stem cells.

The term "antibody", as used herein, refers to an immunoglobulin or a part thereof, and encompasses any polypeptide comprising an antigen-binding site regardless of the source, species of origin, method of production, and characteristics. Antibodies may be comprised of heavy and/or light chains or fragments thereof. Antibodies or antigen-binding fragments, variants, or derivatives thereof of the invention include, but are not limited to, polyclonal, monoclonal, multispecific, human, humanized, primatized, or chimeric antibodies, single chain antibodies, epitope-binding fragments, e.g., Fab, Fab' and F(ab')₂, Fd, Fvs, single-chain Fvs (scFv), single-chain antibodies, disulfide-linked Fvs (sdFv), fragments comprising either a VL or VH domain, fragments produced by a Fab expression library, and anti-idiotypic (anti-Id) antibodies. ScFv molecules are known in the art and are described, e.g., in U.S. Pat. No. 5,892,019. Immunoglobulin or antibody molecules of the invention can be of any type (e.g., IgG, IgE, IgM, IgD, IgA, and IgY), class (e.g., IgG1, IgG2, IgG3, IgG4, IgA1 and IgA2) or subclass of immunoglobulin molecule.

The term "biologic therapeutic" or "biopharmaceutical", as used herein, refers to any medicinal product manufactured in or extracted from biological sources. Biopharmaceuticals are distinct from chemically synthesized pharmaceutical products. Examples of biopharmaceuticals include vaccines, blood or blood components, allergenics, somatic cells, gene therapies, tissues, recombinant therapeutic proteins, including antibody therapeutics and fusion proteins, and living cells. Biologics can be composed of sugars, proteins or nucleic acids or complex combinations of these substances, or may be living entities such as cells and tissues. Biologics are isolated from a variety of natural sources human, animal or microorganism and may be produced by biotechnology methods and other technologies. Specific examples of biologic therapeutics include, but are not limited to, immunostimulatory agents, T cell growth factors, interleukins, antibodies, fusion proteins and vaccines, such as cancer vaccines.

The term "cancer", as used herein, shall be given its ordinary meaning, as a general term for diseases in which abnormal cells divide without control. In particular, and in the context of the embodiments of the present invention, cancer refers to angiogenesis-related cancer. Cancer cells can invade nearby tissues and can spread through the bloodstream and lymphatic system to other parts of the body. There are several main types of cancer, for example, carcinoma is cancer that begins in the skin or in tissues that line or cover internal organs. Sarcoma is cancer that begins in bone, cartilage, fat, muscle, blood vessels, or other connective or supportive tissue. Leukemia is cancer that starts in blood-forming tissue such as the bone marrow, and causes large numbers of abnormal blood cells to be produced and enter the bloodstream. Lymphoma is cancer that begins in the cells of the immune system.

When normal cells lose their ability to behave as a specified, controlled and coordinated unit, a tumor is formed. Generally, a solid tumor is an abnormal mass of tissue that usually does not contain cysts or liquid areas (some brain tumors do have cysts and central necrotic areas filled with liquid). A single tumor may even have different populations of cells within it, with differing processes that have gone awry. Solid tumors may be benign (not cancerous), or malignant (cancerous). Different types of solid tumors are named for the type of cells that form them. Examples of solid tumors are sarcomas, carcinomas, and lymphomas. Leukemias (cancers of the blood) generally do not form solid tumors.

Representative cancers include, but are not limited to, Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Glioblastoma, Childhood; Glioblastoma, Adult; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland' Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor, among others.

A tumor can be classified as malignant or benign. In both cases, there is an abnormal aggregation and proliferation of cells. In the case of a malignant tumor, these cells behave more aggressively, acquiring properties of increased invasiveness. Ultimately, the tumor cells may even gain the ability to break away from the microscopic environment in which they originated, spread to another area of the body (with a very different environment, not normally conducive to their growth), and continue their rapid growth and division in this new location. This is called metastasis. Once malignant cells have metastasized, achieving a cure is more difficult. Benign tumors have less of a tendency to invade and are less likely to metastasize.

Brain tumors spread extensively within the brain but do not usually metastasize outside the brain. Gliomas are very invasive inside the brain, even crossing hemispheres. They do divide in an uncontrolled manner, though. Depending on their location, they can be just as life threatening as malignant lesions. An example of this would be a benign tumor in the brain, which can grow and occupy space within the skull, leading to increased pressure on the brain.

The term "enriched", as used herein, refers to increasing the total percentage of one or more cytotoxic immune cell types present (e.g., γδ T-cells and/or NK cells) l in a sample, relative to the total percentage of the same one or more cell types prior to enrichment, as disclosed herein. For example, a sample that is "enriched" for a for one or more types of cytotoxic immune cell may comprise between about 10% to 100% of the one or more cytotoxic immune cell types in the sample, whereas the total percentage of one or more of the cytotoxic immune cell types in a sample prior to enrichment was, for example, between 0% and 10%. Preferably, an enriched sample comprises at least 10%, 15%, 20%, 25%, 30%, 35%, 40%, 50%, 60% ,70%, 80%, 90% or 100%, of one or more types of cytotoxic immune cell. Samples may be enriched for one or more cell types using standard techniques, for example, flow cytometry techniques.

The term "highly enriched", as used herein, refers to increasing the total percentage of one or more cytotoxic immune cell types in a sample such that the one or more cytotoxic immune cell types may comprise between at least about 70% to about 100% of the cytotoxic immune cell type in the sample, whereas the total percentage of that same type of cytotoxic immune cell prior to enrichment was, for example, between 0% and 10%. Preferably, a highly enriched sample comprises at least 70%, 75%, 80%, 85%, 90%, 95%, 99% or more of one or more types of cytotoxic immune cell. Samples may be highly enriched for one or more cell types using standard techniques, for example, flow cytometry techniques.

The terms "expanded" and "expansion" as used herein with regard to expansion of one or more cytotoxic immune cells in a sample means to increase in the number of one or more cytotoxic immune cells in a sample by, for example about at least 2-fold, preferably by about 5-fold, preferably by at least 10-fold, preferably about at least 50-fold or more. Expansion of a cytotoxic immune cell population can be accomplished by any number of methods as are known in the art. For example, T-cells can be rapidly expanded using non-specific T-cells receptor stimulation in the presence of feeder lymphocytes and either interleukin-2 (IL-2) or interleukin-15 (IL-15), with IL-2 being preferred. The non-specific T-cells receptor stimulus can include around 30 ng/ml of OKT3, a mouse monoclonal anti-CD3 antibody (available from ORTHO-MCNEIL^{®}, Raritan, N.J.). Alternatively T-cells can be rapidly expanded by stimulation of peripheral blood mononuclear cells (PBMC) *in vitro* with one or more antigens (including antigenic portions thereof, such as epitope(s), or a cell) of the cancer, which can be optionally expressed from a vector, such as an human leukocyte antigen A2 (HLA-A2) binding peptide, e.g., 0.3 µM MART-1:26-35 (27 L) or gp100:209-217 (210M), in the presence of a T-cell growth factor, such as 300 IU/ml IL-2 or IL-15, with IL-2 being preferred.

The term "fusion protein", as used herein, refers to chimeric molecules, which comprise, for example, an immunoglobulin antigen-binding domain with at least one target binding site, and at least one heterologous portion, i.e., a portion with which it is not naturally linked in nature. The amino acid sequences may normally exist in separate proteins that are brought together in the fusion polypeptide or they may normally exist in the same protein but are placed in a new arrangement in the fusion polypeptide. Fusion proteins may be created, for example, by chemical synthesis, or by creating and translating a polynucleotide in which the peptide regions are encoded in the desired relationship.

The term "reducing a cancer" as used herein refers to a reduction in the size or volume of a tumor mass, a decrease in the number of metastasized tumors in a subject, a decrease in the proliferative status (the degree to which the cancer cells are multiplying) of the cancer cells, and the like.

The terms "isolated' and isolated population of cells" as used herein refers to a cell or a plurality of cells removed from the tissue or state in which they are found in a subject. The terms may further include cells that have been separated according to such parameters as, but not limited to, cell surface markers, a reporter marker such as a dye or label.

The term "expressed" or "expression" as used herein refers to the transcription from a gene to give an RNA nucleic acid molecule at least complementary in part to a region of one of the two nucleic acid strands of the gene. The term "expressed" or "expression" as used herein also refers to the translation from said RNA nucleic acid molecule to give a protein, a polypeptide, or a portion or fragment thereof.

The term "promoter" as used herein refers to the DNA sequence that determines the site of transcription initiation from an RNA polymerase. A "promoter-proximal element" may be a regulatory sequence within about 200 base pairs of the transcription start site.

The term "recombinant cell" refers to a cell that has a new combination of nucleic acid segments that are not covalently linked to each other in nature. A new combination of nucleic acid segments can be introduced into an organism using a wide array of nucleic acid manipulation techniques available to those skilled in the art. A recombinant cell can be a single eukaryotic cell, or a single prokaryotic cell, or a mammalian cell. The recombinant cell may harbor a vector that is extragenomic. An extragenomic nucleic acid vector does not insert into the cell's genome. A recombinant cell may further harbor a vector or a portion thereof that is intragenomic. The term "intragenomic" defines a nucleic acid construct incorporated within the recombinant cell's genome.

The terms "recombinant nucleic acid" and "recombinant DNA" as used herein refer to combinations of at least two nucleic acid sequences that are not naturally found in a eukaryotic or prokaryotic cell. The nucleic acid sequences include, but are not limited to, nucleic acid vectors, gene expression regulatory elements, origins of replication, suitable gene sequences that when expressed confer antibiotic resistance, protein-encoding sequences, and the like. The term "recombinant polypeptide" is meant to include a polypeptide produced by recombinant DNA techniques such that it is distinct from a naturally occurring polypeptide either in its location, purity or structure. Generally, such a recombinant polypeptide will be present in a cell in an amount different from that normally observed in nature.

The terms "operably" or "operatively linked" as used herein refer to the configuration of the coding and control sequences so as to perform the desired function. Thus, control sequences operably linked to a coding sequence are capable of effecting the expression of the coding sequence. A coding sequence is operably linked to or under the control of transcriptional regulatory regions in a cell when DNA polymerase will bind the promoter sequence and transcribe the coding sequence into mRNA that can be translated into the encoded protein. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

The terms "heterologous" and "exogenous" as they relate to nucleic acid sequences such as coding sequences and control sequences denote sequences that are not normally associated with a region of a recombinant construct or with a particular chromosomal locus, and/or are not normally associated with a particular cell. Thus, a "heterologous" region of a nucleic acid construct is an identifiable segment of nucleic acid within or attached to another nucleic acid molecule that is not found in association with the other molecule in nature. For example, a heterologous region of a construct tip could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a host cell transformed with a construct, which is not normally present in the host cell, would be considered heterologous for purposes of this invention.

Preferably, the promoter will be modified by the addition or deletion of sequences, or replaced with alternative sequences, including natural and synthetic sequences as well as sequences that may be a combination of synthetic and natural sequences. Many eukaryotic promoters contain two types of recognition sequences: the TATA box and the upstream promoter elements. The former, located upstream of the transcription initiation site, is involved in directing RNA polymerase to initiate transcription at the correct site, while the latter appears to determine the rate of transcription and is upstream of the TATA box. Enhancer elements can also stimulate transcription from linked promoters, but many function exclusively in a particular cell type. Many enhancer/promoter elements derived from viruses, e.g., the SV40, the Rous sarcoma virus (RSV), and CMV promoters are active in a wide array of cell types, and are termed "constitutive" or "ubiquitous." The nucleic acid sequence inserted in the cloning site may have any open reading frame encoding a polypeptide of interest, with the proviso that where the coding sequence encodes a polypeptide of interest, it should lack cryptic splice sites that can block production of appropriate mRNA molecules and/or produce aberrantly spliced or abnormal mRNA molecules.

The termination region that is employed primarily will be one of convenience, since termination regions appear to be relatively interchangeable. The termination region may be native to the intended nucleic acid sequence of interest, or may be derived from another source.

The term "targeted therapy", as used herein, refers to any therapeutic molecule that targets any aspect of the immune system.

The term "vector" as used herein refers to a polynucleotide comprised of single strand, double strand, circular, or supercoiled DNA or RNA. A typical vector may be comprised of the following elements operatively linked at appropriate distances for allowing functional gene expression: replication origin, promoter, enhancer, 5' mRNA leader sequence, ribosomal binding site, nucleic acid cassette, termination and polyadenylation sites, and selectable marker sequences. One or more of these elements may be omitted in specific applications. The nucleic acid cassette can include a restriction site for insertion of the nucleic acid sequence to be expressed. In a functional vector the nucleic acid cassette contains the nucleic acid sequence to be expressed including translation initiation and termination sites.

A vector is constructed so that the particular coding sequence is located in the vector with the appropriate regulatory sequences, the positioning and orientation of the coding sequence with respect to the control sequences being such that the coding sequence is transcribed under the "control" of the control or regulatory sequences. Modification of the sequences encoding the particular protein of interest may be desirable to achieve this end. For example, in some cases it may be necessary to modify the sequence so that it may be attached to the control sequences with the appropriate orientation; or to maintain the reading frame. The control sequences and other regulatory sequences may be ligated to the coding sequence prior to insertion into a vector. Alternatively, the coding sequence can be cloned directly into an expression vector that already contains the control sequences and an appropriate restriction site that is in reading frame with and under regulatory control of the control sequences.

The term "lentiviral-based vector" as used herein refers to a lentiviral vector designed to operably insert an exogenous polynucleotide sequence into a host genome in a site-specific manner. Lentiviral-based targeting vectors may be based on, but is not limited to, for example, HIV-1, HIV-2, simian immunodeficiency virus (SIV), or feline immunodeficiency virus (FIV). Preferably, the lentiviral-based targeting vector is an HIV-based targeting vector. This vector may comprise all or a portion of the polynucleotide sequence of HIV.

The terms "transformation", "transduction" and "transduction" all denote the introduction of a polynucleotide into a recipient cell or cells.

Combination Drug Resistant Immunotherapy with Immune Checkpoint Inhibitors

A major limitation to chemotherapy treatments for cancer is drug induced immune toxicity which causes the killing of immunocompetent cells and loss of an effective immune system that would otherwise ward off undesirable infections or provide a defense against cancer cells. One strategy to combat the severe toxic effects of chemotherapy would be to selectively genetically modify cytotoxic immunocompetent cells by the introduction of retroviral vectors designed to express cDNA sequences that confer drug resistance, which can actively target those cancer cells able to resist the simultaneous administration of a chemotherapeutic agent.

Immune checkpoint proteins regulate T cell function in the immune system. T cells play a central role in cell-mediated immunity. Checkpoint proteins interact with specific ligands that send a signal into the T cell and essentially switch off or inhibit T cell function. Cancer cells take advantage of this system by driving high levels of expression of checkpoint proteins on their surface that results in control of the T cells expressing checkpoint proteins on the surface of T cells that enter the tumor microenvironment, thus suppressing the anticancer immune response. As such, inhibition of checkpoint proteins would result in restoration of T cell function and an immune response to the cancer cells. Examples of checkpoint proteins include, but are not limited to CTLA-4, PDL1 (B7-H1, CD274), PDL2 (B7-DC, CD273), PD1, B7-H3 (CD276), B7-H4 (B7-S1, B7x, VCTN1), BTLA (CD272), HVEM, TIM3 (HAVcr2), GAL9, LAG3 (CD223), VISTA, KIR, 2B4 (CD244; belongs to the CD2 family of molecules and is expressed on all NK, γδ, and memory CD8⁺ (αβ) T cells), CD160 (also referred to as BY55), CGEN-15049, CHK 1 and CHK2 kinases, OX40, A2aR and various B-7 family ligands.

Programmed cell death protein 1 (PD-1) is a 288 amino acid cell surface protein molecule that is expressed on T cells and pro-B cells and plays a role in their fate/differentiation. PD-1 has two ligands, PD-L1 and PD-L2, which are members of the B7 family. PD-L1 protein is upregulated on macrophages and dendritic cells (DC) in response to LPS and GM-CSF treatment, and on T cells and B cells upon TCR and B cell receptor signaling. PD-1 negatively regulates T cell responses.

PD-1 plays a role in tumor-specific escape from immune surveillance. It has been demonstrated that PD-1 is highly expressed in tumor-specific cytotoxic T lymphocytes (CTLs) in both chronic myelogenous leukemia (CML) and acute myelogenous leukemia (AML). PD-1 is also up-regulated in melanoma infiltrating T lymphocytes (TILs) [Dotti (2009) Blood 114 (8): 1457-58]. Tumors have been found to express the PD-1 ligands PDL-1 and PDL-2. For example, PDL-1 is found in glioblastoma, especially in mesenchymal subtype. (Nat Rev Neurol 2015: 11:504-515). The expression of PDL-1 and PDL-2 by tumors, combined with the up-regulation of PD-1 in CTLs, may be a contributory factor in the loss in T cell functionality and the inability of CTLs to mediate an effective anti-tumor response. Researchers have shown that in mice chronically infected with lymphocytic choriomeningitis virus (LCMV), administration of anti-PD-1 antibodies blocked PD-1-PDL interaction and was able to restore some T cell functionality (proliferation and cytokine secretion), and lead to a decrease in viral load (Barber et al (2006) Nature 439 (9): 682-687).

Tumor cells themselves expresses PD-L1 and are thought to limit T cell responses via this mechanism. It has further been shown that inhibition of PD-1 results in expansion of effector T cells and restriction of T regulatory cell population in B16 melanoma models. Blockade of PD-1, CTLA-4, or IDO (indoleamine 2,3-dioxygenase) restores IL-2 production and allows for increased proliferation of CD8+ T cells present in the tumor microenvironment. It has been shown that anti-PDL1 treatment rescues and allows expansion of antigen-specific vaccine-generated CD8+ T cells to reject tumor. These data suggest that PD-1/PD-L1 axis regulates activated tumor-specific T cells.

Clinical trials in melanoma, non-small cell lung cancer, and renal cell carcinoma have shown anti-tumor responses in some patients with anti-PD-1 blockade. Significant benefits with PD-1 inhibition in cases of advanced melanoma, non-small-cell lung, prostate, renal-cell, bladder, and colorectal cancer have also been described. Studies in murine models have applied this evidence to glioma therapy. A decrease in tumor-infiltrating Tregs and increased survival when combinatorial treatment of IDO, CTLA-4, and PD-L1 inhibitors was administered has been described.

There are several PD-1 inhibitors currently being tested in clinical trials. CT-01 1 is a humanized IgG1 monoclonal antibody against PD-1. A phase II clinical trial in subjects with diffuse large B-cell lymphoma (DLBCL) who have undergone autologous stem cell transplantation was recently completed. Preliminary results demonstrated that 70% of subjects were progression-free at the end of the follow-up period, compared with 47% in the control group, and 82% of subjects were alive, compared with 62% in the control group. This trial determined that CT-011 not only blocks PD-1 function, but it also augments the activity of natural killer cells, thus intensifying the antitumor immune response.

BMS 936558 is a fully human IgG4 monoclonal antibody targeting PD-1 agents. In a phase I trial, biweekly administration of BMS-936558 in subjects with advanced, treatment-refractory malignancies showed durable partial or complete regressions. The most significant response rate was observed in subjects with melanoma (28%) and renal cell carcinoma (27%), but substantial clinical activity was also observed in subjects with non-small cell lung cancer (NSCLC), and some responses persisted for more than a year. It was also relatively well tolerated; grade≧3 adverse events occurred in 14% of subjects.

BMS 936559 is a fully human IgG4 monoclonal antibody that targets the PD-1 ligand PD-L1. Phase I results showed that biweekly administration of this drug led to durable responses, especially in subjects with melanoma. Objective response rates ranged from 6% to 17% depending on the cancer type in subjects with advanced-stage NSCLC, melanoma, RCC, or ovarian cancer, with some subjects experiencing responses lasting a year or longer.

MK 3475 is a humanized IgG4 anti-PD-1 monoclonal antibody in phase I development in a five-part study evaluating the dosing, safety, and tolerability of the drug in subjects with progressive, locally advanced, or metastatic carcinoma, melanoma, or NSCLC.

MPDL 3280A is a monoclonal antibody, which also targets PD-L1, undergoing phase I testing in combination with the BRAF inhibitor vemurafenib in subjects with BRAF V600-mutant metastatic melanoma and in combination with bevacizumab, which targets vascular endothelial growth factor receptor (VEGFR), with or without chemotherapy in subjects with advanced solid tumors.

AMP 224 is a fusion protein of the extracellular domain of the second PD-1 ligand, PD-L2, and IgG1, which has the potential to block the PD-L2/PD-1 interaction. AMP-224 is currently undergoing phase I testing as monotherapy in subjects with advanced cancer.

Medi 4736 is an anti-PD-L1 antibody in phase I clinical testing in subjects with advanced malignant melanoma, renal cell carcinoma, NSCLC, and colorectal cancer.

CTLA4 (cytotoxic T-lymphocyte-associated protein), is a protein receptor that down regulates the immune system. CTLA4 is found on the surface of T cells, which lead the cellular immune attack on antigens. The T cell attack can be turned on by stimulating the CD28 receptor on the T cell. The T cell attack can be turned off by stimulating the CTLA4 receptor. A first-in-class immunotherapy, ipilimumab (YERVOY^{®}), a monoclonal antibody that targets CTLA-4 on the surface of T cells, was for the treatment of melanoma.

Disclosed herein is a method of treating cancer in a subject in need thereof comprising administering to the subject a therapeutic agent in combination with a drug-resistant immunocompetent cell and an immune checkpoint inhibitor. Preferably, the checkpoint inhibitor is a biologic therapeutic or a small molecule. Preferably, the checkpoint inhibitor is a monoclonal antibody, a humanized antibody, a fully human antibody, a fusion protein or a combination thereof. Preferably, the checkpoint inhibitor inhibits a checkpoint protein which may be CTLA-4, PDL1, or PD1. Preferably, the checkpoint inhibitor interacts with a ligand of a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, OX40, A2aR, B-7 family ligands or a combination thereof.

The therapeutic agent is a chemotherapeutic agent.

Preferably, the drug-resistant immunocompetent cell, therapeutic agent, and checkpoint inhibitor are administered simultaneously, or sequentially in any order, either singly, or one followed by the other two simultaneously, or two simultaneously followed by the third. Preferably, the drug-resistant immunocompetent cell is administered prior to the therapeutic agent and the immune checkpoint inhibitor. Preferably, the checkpoint inhibitor is a PD-1 inhibitor or a CTLA-4 inhibitor.

The population of cytotoxic immune cells used in the invention comprises γδ T-cells. Preferably the population of cytotoxic immune cells comprises about 10%, 20%, 30%, 40%, 50%, 60%, 70%, 75%, 80%, 85%, 90%, 95% or 100% γδ T-cells. Preferably the population of cytotoxic immune cells comprises about 50% to about 95% γδ T-cells. Preferably the population of cytotoxic immune cells used in the invention comprises γδ T-cells and natural killer (NK) cells. Preferably the population of cytotoxic immune cells comprises about 1%, 5%, 10%, 15%, 20%, 25%, 30%, 35%, 40%, 45%, 50%, or more of NK cells. Preferably the population of cytotoxic immune cells comprises about 5% to about 25% of NK cells.

Preferably, the isolated compositions of cytotoxic immune cells used in the invention comprise γδ T-cells, NK cells or any combination thereof and optionally further comprise other immunocompetent cells including but not limited to: monocytes, macrophages and dendritic cells wherein greater than about 50% or more of the cytotoxic immune cells present in the composition express a polypeptide that confers resistance to a chemotherapy agent. Preferably the isolated compositions of cytotoxic immune cells used in accordance with the invention comprises γδ T-cellswherein at least about 50% or more of the γδ T-cells express a polypeptide that confers resistance to a chemotherapy agent. Preferably, the polypeptide that confers resistance to a chemotherapy agent is O⁶ methylguanine DNA methyltransferase (MGMT), a drug resistant variant of dihydrofolate reductase (L22Y-DHFR), thymidylate synthase, multiple drug resistance-1 protein (MDR1).

Disclosed herein are methods of treating cancer in a subject comprising: administering to a subject in need thereof, an immune checkpoint inhibitor in combination with a chemotherapy agent and a chemotherapy resistant composition of immune competent cells comprising γδ T-cells, NK cells or any combination thereof and further optionally comprising other immunocompetent cells including but not limited to: monocytes, macrophages and dendritic cells that are genetically engineered to express a polypeptide that confers resistance to the chemotherapy agent. The generation and expansion of compositions comprising drug-resistant γδ T-cells, NK cells or any combination thereof *ex vivo* can allow, in this setting, for administration of immunocompetent cell-based therapy concurrently with chemotherapy and checkpoint inhibitors, potentially improving tumor clearance while anti-tumor immunity is established and maintained, which might possibly lead to long-term tumor clearance. Preferably, the immunocompetent cells are genetically engineered to express at least one, at least two, at least three or more different tumor recognition moieties wherein each tumor recognition moiety recognizes a tumor antigen.

Disclosed herein are methods of treating cancers, and in particular cancerous tumors. The invention combines the use of chemotherapeutic agents that can kill or reduce the proliferation of cancerous cells, with immunotherapy and immune checkpoint inhibitors to effectively eliminate those cancerous cells that develop drug resistance or otherwise escape the chemotherapeutic agent. The invention provides for isolating cytotoxic immune cells, including, but not limited to, γδ T-cells either from a patient to be treated or from another source, as described, for example, by Lamb L. S. in U.S. Pat. No. 7,078,034. The isolated cells may then be transfected with a nucleic acid vector comprising a heterologous nucleotide sequence encoding a polypeptide that confers resistance to a selected chemotherapeutic agent to the cell. The patient in need of treatment for a cancer, and in particular a tumor, may then receive a dose, or doses, of the transfected T-cells before, after or with the chemotherapeutic agent and the checkpoint inhibitor. The agent itself, while intended to be toxic to the targeted cancer cells, and will reduce the proliferation and viability of the cells, may also induce the microenvironment of the tumor to reduce local tumor-derived immunosuppression and improve migration of cells into the tumor as well as formation on the cell surface of the cancer cells of stress-related proteins. Transfected γδ T-cells, for example, have the characteristic of being able to recognize and therefore target such stress-related ligands, thereby specifically or preferentially targeting the cancer cells.

Preferably, the genetically modified cytotoxic immune cells comprising γδ T-cells may be delivered to the targeted tumor directly by such as, but not limited to, direct injection into the tumor mass, delivery to a blood vessel entering the tumor mass, or a combination of both. For example, it is contemplated that the cells may be delivered to a glioblastoma mass in the brain of a patient by direct implantation through a cannulated needle or catheter inserted into the tumor mass, intracavity post-resection, intraperitoneal or intraventricular.

In some protocols that combine immunotherapy and chemotherapy to treat cancers in a subject human or animal patient, cytokines (IL-2, IL-12, GM-CSF, and the like) may be delivered to a patient to boost the formation of cytotoxic lymphocytes. In others, an anti-CD137 specific antibody may be employed. CD137 is a member of the tumor necrosis factor (TNF)/nerve growth factor (NGF) family of receptors and is expressed by activated T- and B-lymphocytes and monocytes; its ligand has been found to play an important role in the regulation of immune responses. An anti-CD137 monoclonal antibody can specifically bind to CD137-expressing immune cells such as activated T-cells and freshly isolated mouse dendritic cells (DCs), thereby stimulating an immune response, in particular of a cytotoxic T cell response, against tumor cells.

Disclosed herein are methods for treating cancer in a patient, comprising the steps of: obtaining a population of cytotoxic immune cells, where the isolated cytotoxic immune cells have been genetically modified to be resistant to a therapeutic agent; administering to a patient in need thereof, an effective amount of the therapeutic agent; administering to the patient a population of isolated genetically modified cytotoxic immune cells, whereupon the cytotoxic immune cells are delivered to the tumor; and administering to the patient an effective amount of an immune checkpoint inhibitor, thereby reducing the cancer in the patient.

Preferably, the cytotoxic immune cells are γδ T-cells. Preferably the cytotoxic immune cells are γδ T-cells and natural killer (NK) cells. Preferably the cytotoxic immune cells are γδ T-cells, NK cells and may further include other immunocompetent cells including, but not limited to: monocytes, macrophages and dendritic cells. Preferably, the cytotoxic immune cells can be isolated from the patient having the cancer. Preferably, the cytotoxic immune cells may be isolated from a source other than the patient with cancer.

Preferably, the cytotoxic immune cells are γδ T-cells derived from human induced pluripotent stem cells (hiPSCs). Preferably, the cytotoxic immune cells are γδ T-cells and NK cells derived from human induced pluripotent stem cells (hiPSCs). Preferably the cytotoxic immune cells are derived from human induced pluripotent stem cells (hiPSCs) and are γδ T-cells, NK cells and may further include other immunocompetent cells including, but not limited to: monocytes, macrophages and dendritic cells. Preferably, the pluripotent stem cells can be isolated from the patient having the cancer. Preferably, the pluripotent stem cells may be isolated from a source other than the patient with cancer.

Preferably, the therapeutic agent is characterized by a cell developing resistance to said therapeutic agent when the cell receives a heterologous nucleic acid, and wherein the heterologous nucleic acid is expressed in the cell. Preferably, the therapeutic agent can be a cytotoxic chemotherapeutic agent selected from the group consisting of:
alkylating agents (e.g., cyclophosphamide, ifosfamide); metabolic antagonists (e.g., methotrexate (MTX), 5-fluorouracil or derivatives thereof); DNA demethylating agents (also known as antimetabolites; e.g., azacitidine); a substituted nucleotide; a substituted nucleoside; antitumor antibiotics (e.g., mitomycin, adriamycin); plant-derived antitumor agents (e.g., vincristine, vindesine, TAXOL^{®}, paclitaxel, abraxane); cisplatin; carboplatin; etoposide; and the like. Such agents may further include, but are not limited to, the anti-cancer agents trimethotrexate (TMTX); temozolomide; raltitrexed; S-(4-Nitrobenzyl)-6-thioinosine (NBMPR); 6-benzyguanidine (6-BG); nitrosoureas [e.g., bis-chloronitrosourea (BCNU; carmustine), lomustine (CCNU) +/- Procarbazine and Vincristine (PCV regimen), fotemustine]; cytarabine; camptothecin; and a therapeutic derivative of any thereof.

Preferably, the step of obtaining a population of cytotoxic immune cells genetically modified to be resistant to a therapeutic agent comprises: obtaining from a subject such as a human subject or animal subject a population of cytotoxic immune cells, for example by obtaining a biological sample from the subject including but not limited to a blood or tissue sample including a tumor biopsy. The sample may optionally be enriched for cytotoxic immune cells and other immunocompetent cells and/or the cells present in the sample may optionally be expanded to increase the population of the cells present in the sample. The cells are preferably stably transfected or gene edited with a vector comprising a heterologous nucleic acid sequence operably linked to a promoter, wherein the heterologous nucleic acid sequence encodes a polypeptide conferring to the cell resistance to one or more therapeutic agents such as a chemotherapeutic agent. Preferably, the population of stably transfected cytotoxic immune cells can be viably maintained.

Preferably, the therapeutic agent can be trimethotrexate or methotrexate, and the heterologous nucleic acid sequence encodes dihydrofolate reductase, or a derivative thereof. Preferably, the therapeutic agent can be temozolomide, or a therapeutically active derivative thereof, and the heterologous nucleic acid sequence may encode O⁶ methylguanine DNA methyltransferase, or a derivative thereof. Preferably, the chemotherapeutic agent can be cisplatin, doxorubicin or paclitaxel, or a therapeutically active derivative thereof, and the heterologous nucleic acid sequence may encode multiple drug resistance-1 protein (MDR1), or a derivative thereof.

Preferably, the isolated genetically modified cytotoxic immune cells, the therapeutic agent, and the immune checkpoint inhibitor can be co-administered to the patient. Preferably, the genetically modified cytotoxic immune cells, the therapeutic agent, and the immune checkpoint inhibitor can be sequentially administered to the patient. Preferably, the genetically modified cytotoxic immune cells, the therapeutic agent, and the immune checkpoint inhibitor can be concomitantly administered to the patient. Preferably, the genetically modified cytotoxic immune cells are administered to the patient directly into the tumor or to a blood vessel proximal and leading into the tumor. Preferably, the tumor is a glioblastoma.

Preferably, the immune checkpoint inhibitor can be a biologic therapeutic or a small molecule. Preferably, the checkpoint inhibitor is a monoclonal antibody, a humanized antibody, a fully human antibody, a fusion protein, an antigen-binding fragment or a combination thereof. In the present invention, the checkpoint inhibitor inhibits a checkpoint protein which may be CTLA-4, PDL1 or PD1. The checkpoint inhibitor may interact with a ligand of a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, OX40, B-7 family ligands or a combination thereof. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), OPDIVO^{®}/Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and YERVOY^{®}/ipilimumab (anti-CTLA-4 checkpoint inhibitor). Checkpoint protein ligands include, but are not limited to PD-L1, PD-L2, B7-H3, B7-H4, CD28, CD86 and TIM-3.

Preferably, the present invention uses a class of immune checkpoint inhibitor drugs that inhibit CTLA-4. Suitable anti-CTLA4 antagonist agents for use in the invention, include, without limitation, anti-CTLA4 antibodies, human anti-CTLA4 antibodies, mouse anti-CTLA4 antibodies, mammalian anti-CTLA4 antibodies, humanized anti-CTLA4 antibodies, monoclonal anti-CTLA4 antibodies, polyclonal anti-CTLA4 antibodies, chimeric anti-CTLA4 antibodies, MDX-010 (ipilimumab), tremelimumab, anti-CD28 antibodies, anti-CTLA4 adnectins, anti-CTLA4 domain antibodies, single chain anti-CTLA4 fragments, heavy chain anti-CTLA4 fragments, light chain anti-CTLA4 fragments, and inhibitors of CTLA4 that agonize the co-stimulatory pathway. Preferably, additional anti-CTLA4 antagonists include, but are not limited to, the following: any inhibitor that is capable of disrupting the ability of CD28 antigen to bind to its cognate ligand, to inhibit the ability of CTLA4 to bind to its cognate ligand, to augment T cell responses via the co-stimulatory pathway, to disrupt the ability of B7 to bind to CD28 and/or CTLA4, to disrupt the ability of B7 to activate the co-stimulatory pathway, to disrupt the ability of CD80 to bind to CD28 and/or CTLA4, to disrupt the ability of CD80 to activate the co-stimulatory pathway, to disrupt the ability of CD86 to bind to CD28 and/or CTLA4, to disrupt the ability of CD86 to activate the co-stimulatory pathway, and to disrupt the co-stimulatory pathway, in general from being activated. This necessarily includes small molecule inhibitors of CD28, CD80, CD86, CTLA4, among other members of the co-stimulatory pathway; antibodies directed to CD28, CD80, CD86, CTLA4, among other members of the co-stimulatory pathway; antisense molecules directed against CD28, CD80, CD86, CTLA4, among other members of the co-stimulatory pathway; adnectins directed against CD28, CD80, CD86, CTLA4, among other members of the co-stimulatory pathway, RNAi inhibitors (both single and double stranded) of CD28, CD80, CD86, CTLA4, among other members of the co-stimulatory pathway, among other anti-CTLA4 antagonists.

Preferably, immunostimulatory agents, T cell growth factors and interleukins may be used in combination with checkpoint inhibitors and drug-resistant immunotherapy. Immunostimulatory agents are substances (drugs and nutrients) that stimulate the immune system by inducing activation or increasing activity of any of its components. Immunostimulants include, but are not limited to, bacterial vaccines, colony stimulating factors, interferons, interleukins, other immunostimulants, therapeutic vaccines, vaccine combinations and viral vaccines. T cell growth factors are proteins that stimulate the proliferation of T cells. Examples of T cell growth factors include Il-2, IL-7, IL-15, IL-17, IL21 and IL-33.

Preferably the invention use compositions comprising at least one checkpoint inhibitor, and an isolated population of cytotoxic immune cells comprising γδ T-cellswherein greater than about 50% of the population of cytotoxic immune cells expresses a polypeptide that confers resistance to a chemotherapy agent. Preferably the composition comprises at least one checkpoint inhibitor, and an isolated population of cytotoxic immune cells comprising γδ T-cells and NK cells wherein about 50% to about 95% of the population of cytotoxic immune cells are γδ T-cells and wherein about 5% to about 25% of the population of cytotoxic immune cells are NK cells.

Preferably, the therapeutic, immune checkpoint inhibitor, biologic therapeutic or pharmaceutical composition as disclosed herein can be administered to an individual by various routes including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin using, for example, a skin patch or transdermal iontophoresis, respectively. The therapeutic, checkpoint inhibitor, biologic therapeutic or pharmaceutical composition also can be administered to the site of a pathologic condition, for example, intravenously or intra-arterially into a blood vessel supplying a tumor.

Preferably, the immune checkpoint inhibitor is administered in less than 0.0001 mg/kg, 0.0001-0.001 mg/kg, 0.001-0.01 mg/kg, 0.01-0.05 mg/kg, 0.05-0.1 mg/kg, 0.1-0.2 mg/kg, 0.2-0.3 mg/kg, 0.3-0.5 mg/kg, 0.5-0.7 mg/kg, 0.7-1 mg/kg, 1-2 mg/kg, 2-3 mg/kg, 3-4 mg/kg, 4-5 mg/kg, 5-6 mg/kg, 6-7 mg/kg, 7-8 mg/kg, 8-9 mg/kg, 9-10 mg/kg, at least 10 mg/kg, or any combination thereof doses. Preferably, the checkpoint inhibitor is administered at least once a week, at least twice a week, at least three times a week, at least once every two weeks, or at least once every month or multiple months. Preferably, the checkpoint inhibitor is administered as a single dose, in two doses, in three doses, in four doses, in five doses, or in 6 or more doses.

Disclosed herein are systems for treating a cancer in a patient comprising a cytotoxic therapeutic agent having the characteristics of inhibiting the survival of a cancer cell, an isolated population of cytotoxic immune cells, where the cytotoxic immune cells genetically modified to be resistant to the therapeutic agent, and an immune checkpoint inhibitor. Preferably, the population of cytotoxic immune cells comprises γδ T-cells, NK cells or any combination thereof. Preferably the population of cytotoxic immune cells comprises γδ T-cells and natural killer cells and optionally comprises other immunocompetent cells. Preferably, the population of cytotoxic immune cells may comprise a heterologous nucleic acid sequence operably linked to a promoter, where the heterologous nucleic acid sequence encodes a polypeptide that when expressed in a cell confers resistance to the therapeutic agent to the cell. Preferably, the therapeutic agent is a cytotoxic chemotherapeutic agent selected from the group consisting of: an alkylating agent (e.g., cyclophosphamide, ifosfamide); a metabolic antagonist (e.g., methotrexate (MTX), 5-fluorouracil or derivatives thereof); a DNA demethylating agent (also known as antimetabolites; e.g., azacitidine); a substituted nucleotide; a substituted nucleoside; an antitumor antibiotic (e.g., mitomycin, adriamycin); a plant-derived antitumor agent (e.g., vincristine, vindesine, TAXOL^{®}, paclitaxel, abraxane); cisplatin; carboplatin; etoposide; and the like. Such agents may further include, but are not limited to, the anti-cancer agents trimethotrexate (TMTX); temozolomide; raltitrexed; S-(4-Nitrobenzyl)-6-thioinosine (NBMPR); 6-benzyguanidine (6-BG); nitrosoureas [e.g., bis-chloronitrosourea (BCNU; carmustine), lomustine (CCNU) +/- Procarbazine and Vincristine (PCV regimen), fotemustine]; cytarabine; camptothecin; and a therapeutic derivative of any thereof.
Preferably, the therapeutic agent is trimethotrexate or methotrexate, and the heterologous nucleic acid sequence encodes dihydrofolate reductase, or a derivative thereof. Preferably, the therapeutic agent is temozolomide, or a therapeutically agent derivative thereof, and the heterologous nucleic acid sequence encodes O⁶ methylguanine DNA methyltransferase (MGMT), or a derivative thereof. Preferably, the therapeutic agent is a nitrosourea, or a therapeutically agent derivative thereof, and the heterologous nucleic acid sequence encodes O⁶ methylguanine DNA methyltransferase (MGMT), or a derivative thereof.

Preferably, the immune checkpoint inhibitor is a biologic therapeutic or a small molecule. Preferably, the checkpoint inhibitor is a monoclonal antibody, a humanized antibody, a fully human antibody, a fusion protein, an antigen-binding fragment or a combination thereof. The checkpoint inhibitor inhibits a checkpoint protein which may be CTLA-4, PDL1 or PD1. the checkpoint inhibitor may interact with a ligand of a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, OX40, B-7 family ligands or a combination thereof. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), OPDIVO^{®}/Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and YERVOY^{®}/ipilimumab (anti-CTLA-4 checkpoint inhibitor). Checkpoint protein ligands include, but are not limited to PD-L1, PD-L2, B7-H3, B7-H4, CD28, CD86 and TIM-3.

Preferably, the present invention uses a class of checkpoint inhibitor drugs that inhibit CTLA-4. Suitable anti-CTLA4 antagonist agents for use in the invention, include, without limitation, anti-CTLA4 antibodies, human anti-CTLA4 antibodies, mouse anti-CTLA4 antibodies, mammalian anti-CTLA4 antibodies, humanized anti-CTLA4 antibodies, monoclonal anti-CTLA4 antibodies, polyclonal anti-CTLA4 antibodies, chimeric anti-CTLA4 antibodies, MDX-010 (ipilimumab), tremelimumab, anti-CD28 antibodies, anti-CTLA4 adnectins, anti-CTLA4 domain antibodies, single chain anti-CTLA4 fragments, heavy chain anti-CTLA4 fragments, light chain anti-CTLA4 fragments, and inhibitors of CTLA4 that agonize the co-stimulatory pathway.

Preferably, additional anti-CTLA4 antagonists include, but are not limited to, the following: any inhibitor that is capable of disrupting the ability of CD28 antigen to bind to its cognate ligand, to inhibit the ability of CTLA4 to bind to its cognate ligand, to augment T cell responses via the co-stimulatory pathway, to disrupt the ability of B7 to bind to CD28 and/or CTLA4, to disrupt the ability of B7 to activate the co-stimulatory pathway, to disrupt the ability of CD80 to bind to CD28 and/or CTLA4, to disrupt the ability of CD80 to activate the co-stimulatory pathway, to disrupt the ability of CD86 to bind to CD28 and/or CTLA4, to disrupt the ability of CD86 to activate the co-stimulatory pathway, and to disrupt the co-stimulatory pathway, in general from being activated. This necessarily includes small molecule inhibitors of CD28, CD80, CD86, CTLA4, among other members of the co-stimulatory pathway; antibodies directed to CD28, CD80, CD86, CTLA4, among other members of the co-stimulatory pathway; antisense molecules directed against CD28, CD80, CD86, CTLA4, among other members of the co-stimulatory pathway; adnectins directed against CD28, CD80, CD86, CTLA4, among other members of the co-stimulatory pathway, RNAi inhibitors (both single and double stranded) of CD28, CD80, CD86, CTLA4, among other members of the co-stimulatory pathway, among other anti-CTLA4 antagonists.

Preferably, immunostimulatory agents, T cell growth factors and interleukins may be used. Immunostimulatory agents are substances (drugs and nutrients) that stimulate the immune system by inducing activation or increasing activity of any of its components. Immunostimulants include, but are not limited to, bacterial vaccines, colony stimulating factors, interferons, interleukins, other immunostimulants, therapeutic vaccines, vaccine combinations and viral vaccines. T cell growth factors are proteins that stimulate the proliferation of T cells. Examples of T cell growth factors include Il-2, IL-7, IL-15, IL-17, IL21 and IL-33.

Preferably, the therapeutic, checkpoint inhibitor, biologic therapeutic or pharmaceutical composition as disclosed herein can be administered to an individual by various routes including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin using, for example, a skin patch or transdermal iontophoresis, respectively. The therapeutic, checkpoint inhibitor, biologic therapeutic or pharmaceutical composition also can be administered to the site of a pathologic condition, for example, intravenously or intra-arterially into a blood vessel supplying a tumor.

Preferably, the total amount of an agent to be administered in practicing the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary.

Preferably, the checkpoint inhibitor is administered in less than 0.0001 mg/kg, 0.0001-0.001 mg/kg, 0.001-0.01 mg/kg, 0.01-0.05 mg/kg, 0.05-0.1 mg/kg, 0.1-0.2 mg/kg, 0.2-0.3 mg/kg, 0.3-0.5 mg/kg, 0.5-0.7 mg/kg, 0.7-1 mg/kg, 1-2 mg/kg, 2-3 mg/kg, 3-4 mg/kg, 4-5 mg/kg, 5-6 mg/kg, 6-7 mg/kg, 7-8 mg/kg, 8-9 mg/kg, 9-10 mg/kg, at least 10 mg/kg, or any combination thereof doses. Preferably, the checkpoint inhibitor is administered at least once a week, at least twice a week, at least three times a week, at least once every two weeks, or at least once every month or multiple months. Preferably, the checkpoint inhibitor is administered as a single dose, in two doses, in three doses, in four doses, in five doses, or in 6 or more doses.

Disclosed herein are systems for treating a glioblastoma in a patient comprising a therapeutic agent having the characteristics of inhibiting the survival of a cancer cell and inducing a stress protein in the cancer cell, an isolated population of cytotoxic immune cells, wherein said cytotoxic immune cells comprise γδ T-cells, NK cells, or any combination thereof and further optionally comprise other immunocompetent cells wherein the population of cytotoxic immune cells have been genetically modified to be resistant to the therapeutic agent, and a checkpoint inhibitor. Preferably, the population cytotoxic immune cells comprise a heterologous nucleic acid sequence operably linked to a promoter, wherein the heterologous nucleic acid sequence encodes a polypeptide that when expressed in a cell confers resistance to the therapeutic agent to the cell.

Preferably, the therapeutic agent is a cytotoxic chemotherapeutic agent selected from the group consisting of: an alkylating agent, a metabolic antagonist, an antitumor antibiotic, and a plant-derived antitumor agent.

Preferably, the therapeutic agent is selected from the group consisting of: trimethotrexate (TMTX), methotrexate (MTX), temozolomide, reltritrexed, S-(4-Nitrobenzyl)-6-thioinosine (NBMPR), camptothecin, 6-benzylguanidine, cytarabine, and a therapeutic derivative of any thereof.

Preferably, the therapeutic agent is trimethotrexate or methotrexate, and the heterologous nucleic acid sequence encodes dihydrofolate reductase, or a derivative thereof.

Preferably, the immune checkpoint inhibitor can be a biologic therapeutic or a small molecule. Preferably, the checkpoint inhibitor is a monoclonal antibody, a humanized antibody, a fully human antibody, a fusion protein, an antigen-binding fragment or a combination thereof. The checkpoint inhibitor inhibits a checkpoint protein which may be CTLA-4, PDL1 orPD1. The checkpoint inhibitor may interact with a ligand of a checkpoint protein which may be CTLA-4, PDL1, PDL2, PD1, B7-H3, B7-H4, BTLA, HVEM, TIM3, GAL9, LAG3, VISTA, KIR, 2B4, CD160, CGEN-15049, CHK 1, CHK2, A2aR, OX40, B-7 family ligands or a combination thereof. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), OPDIVO^{®}/Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and YERVOY^{®}/ipilimumab (anti-CTLA-4 checkpoint inhibitor). Checkpoint protein ligands include, but are not limited to PD-L1, PD-L2, B7-H3, B7-H4, CD28, CD86 and TIM-3.

Preferably, the present invention uses a specific class of immune checkpoint inhibitor drugs that inhibit CTLA-4. Suitable anti-CTLA4 antagonist agents for use in the invention, include, without limitation, anti-CTLA4 antibodies, human anti-CTLA4 antibodies, mouse anti-CTLA4 antibodies, mammalian anti-CTLA4 antibodies, humanized anti-CTLA4 antibodies, monoclonal anti-CTLA4 antibodies, polyclonal anti-CTLA4 antibodies, chimeric anti-CTLA4 antibodies, MDX-010 (ipilimumab), tremelimumab, anti-CD28 antibodies, anti-CTLA4 adnectins, anti-CTLA4 domain antibodies, single chain anti-CTLA4 fragments, heavy chain anti-CTLA4 fragments, light chain anti-CTLA4 fragments, and inhibitors of CTLA4 that agonize the co-stimulatory pathway.

Preferably, additional anti-CTLA4 antagonists include, but are not limited to, the following: any inhibitor that is capable of disrupting the ability of CD28 antigen to bind to its cognate ligand, to inhibit the ability of CTLA4 to bind to its cognate ligand, to augment T cell responses via the co-stimulatory pathway, to disrupt the ability of B7 to bind to CD28 and/or CTLA4, to disrupt the ability of B7 to activate the co-stimulatory pathway, to disrupt the ability of CD80 to bind to CD28 and/or CTLA4, to disrupt the ability of CD80 to activate the co-stimulatory pathway, to disrupt the ability of CD86 to bind to CD28 and/or CTLA4, to disrupt the ability of CD86 to activate the co-stimulatory pathway, and to disrupt the co-stimulatory pathway, in general from being activated. This necessarily includes small molecule inhibitors of CD28, CD80, CD86, CTLA4, among other members of the co-stimulatory pathway; antibodies directed to CD28, CD80, CD86, CTLA4, among other members of the co-stimulatory pathway; antisense molecules directed against CD28, CD80, CD86, CTLA4, among other members of the co-stimulatory pathway; adnectins directed against CD28, CD80, CD86, CTLA4, among other members of the co-stimulatory pathway, RNAi inhibitors (both single and double stranded) of CD28, CD80, CD86, CTLA4, among other members of the co-stimulatory pathway, among other anti-CTLA4 antagonists.

Preferably, immunostimulatory agents, T cell growth factors and interleukins may be used. Immunostimulatory agents are substances (drugs and nutrients) that stimulate the immune system by inducing activation or increasing activity of any of its components. Immunostimulants include, but are not limited to, bacterial vaccines, colony stimulating factors, interferons, interleukins, other immunostimulants, therapeutic vaccines, vaccine combinations and viral vaccines. T cell growth factors are proteins that stimulate the proliferation of T cells. Examples of T cell growth factors include Il-2, IL-7, IL-15, IL-17, IL21 and IL-33.

Preferably, the therapeutic, immune checkpoint inhibitor, biologic therapeutic or pharmaceutical composition as disclosed herein can be administered to an individual by various routes including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin using, for example, a skin patch or transdermal iontophoresis, respectively. The therapeutic, checkpoint inhibitor, biologic therapeutic or pharmaceutical composition also can be administered to the site of a pathologic condition, for example, intravenously or intra-arterially into a blood vessel supplying a tumor.

Preferably, the total amount of an agent to be administered in practicing the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary.

Preferably, the checkpoint inhibitor is administered in less than 0.0001 mg/kg, 0.0001-0.001 mg/kg, 0.001-0.01 mg/kg, 0.01-0.05 mg/kg, 0.05-0.1 mg/kg, 0.1-0.2 mg/kg, 0.2-0.3 mg/kg, 0.3-0.5 mg/kg, 0.5-0.7 mg/kg, 0.7-1 mg/kg, 1-2 mg/kg, 2-3 mg/kg, 3-4 mg/kg, 4-5 mg/kg, 5-6 mg/kg, 6-7 mg/kg, 7-8 mg/kg, 8-9 mg/kg, 9-10 mg/kg, at least 10 mg/kg, or any combination thereof doses. Preferably, the checkpoint inhibitor is administered at least once a week, at least twice a week, at least three times a week, at least once every two weeks, or at least once every month or multiple months. Preferably, the checkpoint inhibitor is administered as a single dose, in two doses, in three doses, in four doses, in five doses, or in 6 or more doses.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt. % to about 5 wt. %, but also include individual concentrations (e.g., 1%, 2%, 3%, and 4%) and the sub-ranges (e.g., 0.5%, 1.1%, 2.2%, 3.3%, and 4.4%) within the indicated range. The term "about" can include ±1%, ±2%, ±3%, ±4%, ±5%, ±6%, ±7%, ±8%, ±9%, or ±10%, or more of the numerical value(s) being modified. In addition, the phrase "about 'x' to 'y'" includes "about 'x' to about 'y'".

Many variations and modifications may be made to the above-described invention. All such modifications and variations are intended to be included herein within the scope of this invention and protected by the following claims.

### EXAMPLES

The following examples are offered by way of illustration and are not to be construed as limiting the invention as claimed in any way.

### Example 1- Cancer Treatment Paradigms

A combination therapy comprising a chemotherapeutic agent, drug-resistant γδ-T cells and/or drug-resistant natural killer cells, and an immune checkpoint inhibitor(s) is used to treat the cancer patient. The chemotherapeutic agent is selected from alkylating agents (e.g., cyclophosphamide, ifosfamide), metabolic antagonists (e.g., methotrexate (MTX), 5-fluorouracil or derivatives thereof), antitumor antibiotics (e.g., mitomycin, adriamycin), plant-derived antitumor agents (e.g., vincristine, vindesine, TAXOL^{®}, paclitaxel, abraxane), cisplatin, carboplatin, etoposide, and the like. Such agents may further include, but are not limited to, the anti-cancer agents trimethotrexate (TMTX), temozolomide, raltitrexed, S-(4-Nitrobenzyl)-6-thioinosine (NBMPR), 6-benzyguanidine (6-BG), bis-chloronitrosourea (BCNU), cytarabine, and camptothecin, or a therapeutic derivative of any thereof. The drug-resistant γδ-T cells and natural killer cells are resistant to the chemotherapeutic agent. The immune checkpoint inhibitor(s) is used alone or in combination with other immune checkpoint inhibitors, and is selected from inhibitors of the immune checkpoint proteins CTLA-4, PDL1 (B7-H1, CD274), PDL2 (B7-DC, CD273), PD1, B7-H3 (CD276), B7-H4 (B7-S1, B7x, VCTN1), BTLA (CD272), HVEM, TIM3 (HAVcr2), GAL9, LAG3 (CD223), VISTA, KIR, 2B4 (CD244; belongs to the CD2 family of molecules and is expressed on all NK, γδ, and memory CD8⁺ (αβ) T cells), CD160 (also referred to as BY55), CGEN-15049, CHK 1 and CHK2 kinases, OX40, A2aR and various B-7 family ligands.

### Evaluation of Immune Therapy Activity in Combination with Chemotherapy

Response assessment criteria include evaluation based upon Response Evaluation Criteria in Solid Tumors (WHO; World Health Organization Offset Publication No. 48, 1979); RECIST (Response Evaluation Criteria in Solid Tumors; J Natl Cancer Inst 2000; 92:205-16; Eisenhauer EA et al., Eur J Cancer 2009;45:228-47); Immune-Related Response Criteria (Jedd WE et al., Clin Cancer Res 2009;15(23),7412-7420); and iRANO (Immunotherapy Response Assessment in Neuro-Oncology; Okada H et al., Lancet Oncol 2015 Nov; 16(15):e534-42).

### Results

Chemotherapy as a combination therapy additionally comprising drug-resistant γδ-T cells (and/or drug-resistant natural killer cells) and an inhibitor(s) of immune checkpoint proteins results in an enhanced or prolonged anti-tumor response; a medically beneficial response; additive or synergistic antitumor activity, when compared with chemotherapy alone, or chemotherapy combined with drug-resistant γδ-T cells (and/or drug-resistant natural killer cells); shrinkage in baseline lesions, without new lesions; tumor regression; durable stable disease with possible steady decline in total tumor burden; response after an increase in total tumor burden; and response in the presence of new lesions. Other clinical findings may include an increase in tumor infiltrating lymphocytes as measured by an increase in tumor size; and long-term survival promotion.

For example, temozolomide (TMZ)-treated tumors express increased numbers of stress ligands on the surface of the tumor cell, such as MICA, MICB, and ULBP1-6. The killing of tumor cells is facilitated by the drug-resistant γδ-T cells and/or drug-resistant natural killer cells whose receptor(s), such as NKG2D, recognizes the tumor-specific stress ligands. However, tumor cells can enzymatically cleave MICA and MICB, releasing soluble MICA and MICB. When bound by effector cell receptors in the plasma, this can lead to down regulation of the receptor expression resulting in immune escape. Chemotherapies such as TMZ have been found to increase cell surface expression of stress ligands on the tumor tissue without significantly increasing the concentration of the soluble ligands in the plasma. Responders to antibody inhibitors of CTLA-4 have demonstrated the ability to produce a humoral response against NKG2D ligands such as MICA. These responses result in a reduction in soluble MICA, which may allow the amplified stress ligand expression on the tumor surface to enhance tumor cell killing by the drug-resistant γδ-T cells and/or drug-resistant natural killer cells.

### Example 2- Analysis of Checkpoint Molecule Expression and Function in Glioma and γδ T cells

Checkpoint molecule expression on GMP manufactured *γδ* T cells and glioblastoma tumor cells was evaluated and the function of enriched *γδ* T cells *in-vitro* was assessed.

### Methods

### Ex vivo expansion of γδ T cells

Human apheresis products collection from healthy donors were purchased from Hemacare (Van Nuys CA). The products were transferred to the UAB Cell Therapy Laboratory GMP facility. For static culture: All manipulations were performed in a Class 100 laminar flow hood surrounded by a Class 10K/ISO 7 classified air flow. The product was diluted v/v with HBSS (Hank's Balanced Salt Solution) and mononuclear cells isolated by density gradient separation on Ficoll (Sigma-Aldrich; St. Louis, MO). Interphase was harvested and washed X2 in HBSS. The cell pellet was resuspended in CTS^{™} OpTmizer^{™} T Cell Expansion serum free media (ThermoFisher Scientific; Waltham, MA) supplemented with 2mM Zoledronic Acid (Novartis; Basel HV) and 100u/mL IL2 (Miltenyi Biotec Ltd; Bergisch Gladbach, Germany). Cells were cultured in standard T150 or T75 flasks. For closed system culture: All operation including preparation of media, stocks and buffers were performed in ISO 7 clean rooms under biosafety cabinets using GMP grade reagents. Automated Ficoll separation of the product and cultivation were carried out in CLINIMACS PRODIGY^{®} (Miltenyi Biotec Ltd; Bergisch Gladbach, Germany) bioreactor using customized cell processing program and TS520 tubing set. Cells were then expanded in the CentriCult-Unit of the CLINIMACS PRODIGY^{®} using a combination of Zoledronate (Zometa; Novartis, Basel) and Interleukin-2 (Miltenyi Biotec Ltd; Bergisch Gladbach, Germany) (ZOL/IL-2), in OpTmizer serum-free culture (Thermo Fisher Scientific; Waltham, MA). The culture was kept at a density of 1-2x 106 cells/ml and supplemented with IL-2 100u/mL every other day until cells harvest at day 13. The process was continuously monitored to determine kinetics of expansion and phenotype in comparison with small-scale culture in flasks at regular intervals. Following 13-day culture, expanded γδ T cells were phenotyped and cytotoxicity determined against standard leukemia cell lines and glioma tumor cells.

### Flow Cytometry Analysis

Multiparametric flow cytometry analysis was used to determine phenotype & functional profile in donor PBMC and cultured products. DuraClone T cell subset (Beckman Coulter) plus anti-PD-1, anti-CTLA-4 and anti-PD-L1 panel were used to monitor expression of checkpoint molecule and γδ T cell enrichment using Flow cytometry. Samples of product were analyzed on culture day 1 and 13. Freshly dissociated PDX derived glioblastoma tumor cells were analyzed for PD-L1 expression using flow cytometry. Single cell suspension of PDX derived glioblastoma cells were blocked with FcR Block (Biolegend) for 15 min, before staining with PD-L1 antibody and respective Isotype (IgG1). The stained cells were acquired on BD fortessa X-20 flow cytometer.

### Flow cytometry based cytotoxicity evaluation

Potency of the cell product was determined using *in-vitro* cytotoxicity assays against K562 cells. Expanded γδ T cells was assessed at day 13 for cytotoxicity on human leukemic cell lines (K562), and tumor cells (JX22T, JX12T AND JX59T) using flow cytometry based cytotoxicity assay. The Basic Cytotoxicity Assay Kit (https:// immunochemistry. com) includes two fluorescent reagents: CFSE and 7-AAD. CFSE, a green fluorescing membrane stain was used to label the target cells (K562). The unstained effector cells are added at increasing effector to target ratio and incubated with the target cells for four hours before adding 7-AAD, a red fluorescing live/dead stain followed by acquisition and analyzes using flow cytometer. The Cytotoxicity is calculated as the percentage of the green target cells which are detected red channel. Formula for % Cytotoxicity = # Dead cells / # Live cells + # Dead cells* 100.

### Results & Discussion

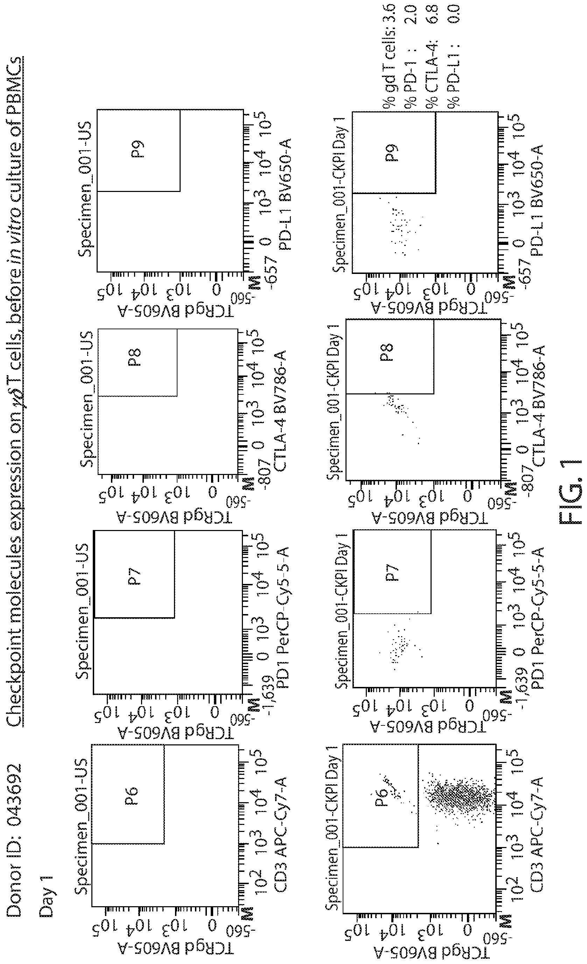

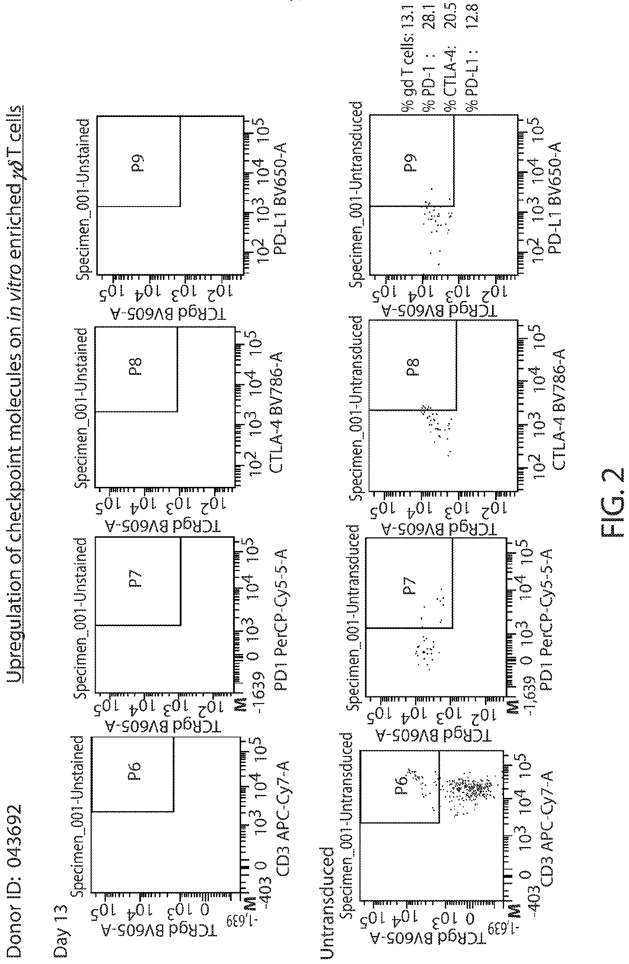

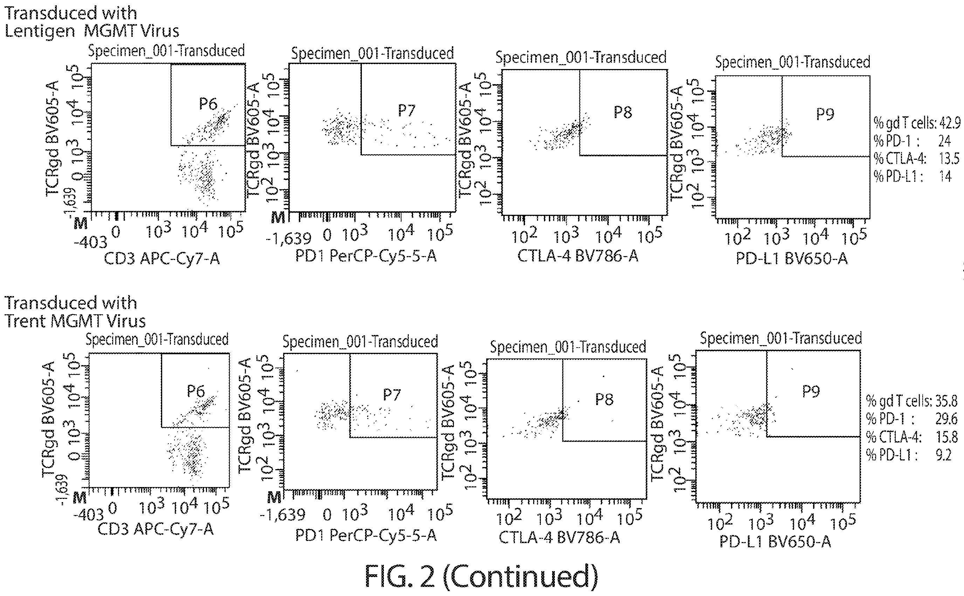

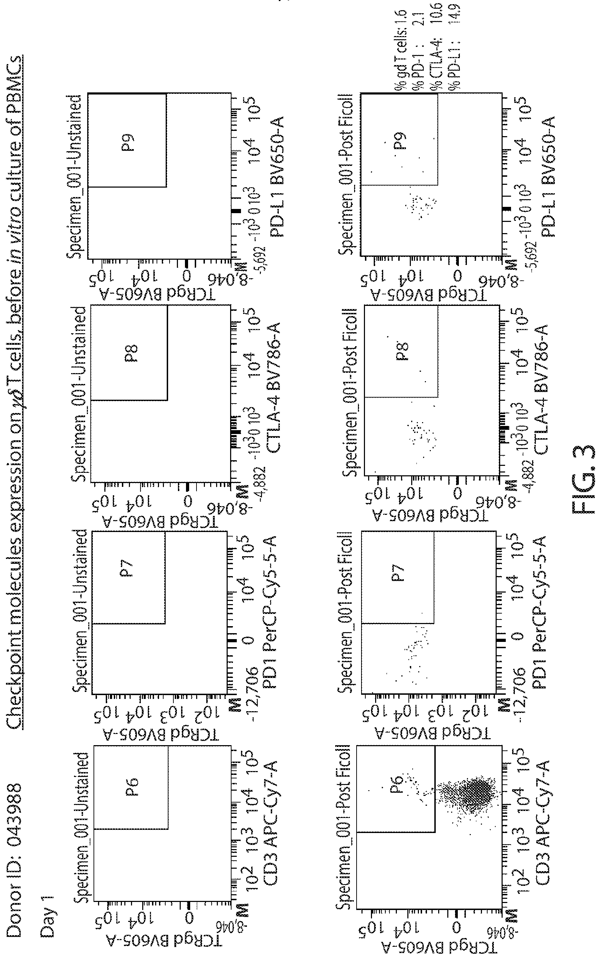

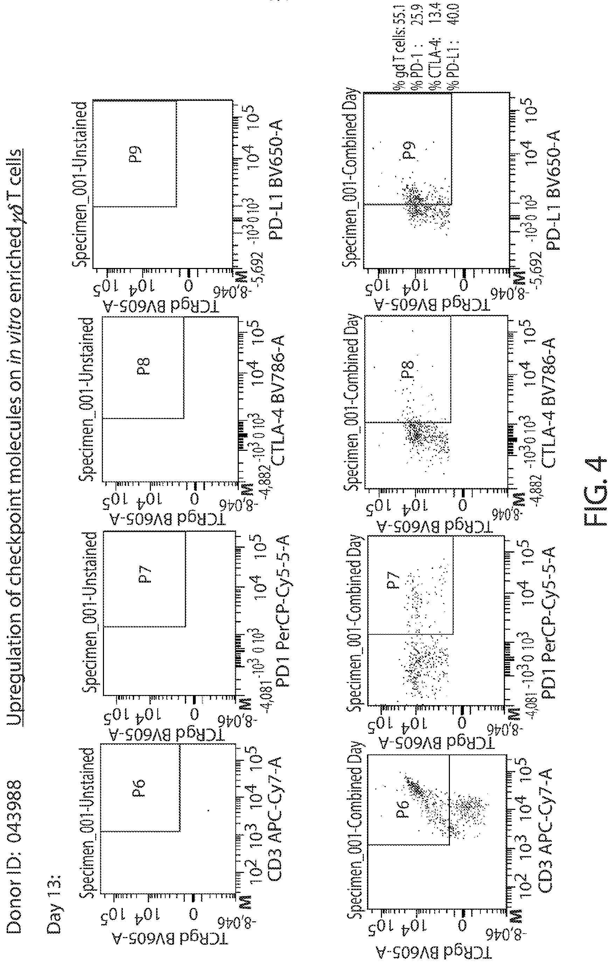

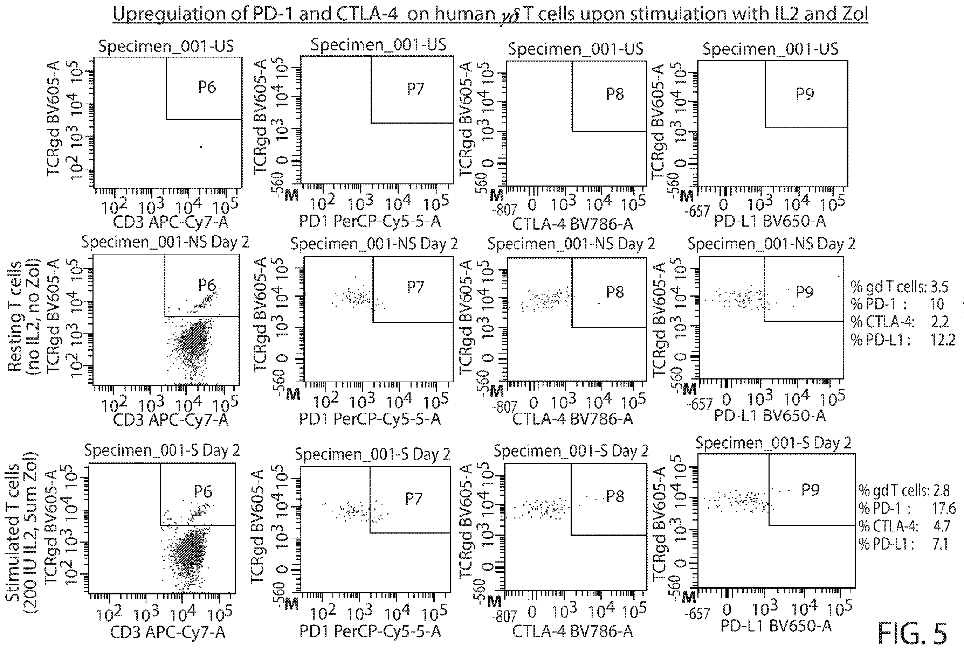

Freshly isolated PBMCs from two healthy donors (donor 1: 043692; donor 2: 043988) were used for expansion of γδ T cells *in vitro.* Upon examining checkpoint expression by flow cytometry, we noted upregulation of PD-1 and CTLA-4 and PD-L1 in expanded *γδ* T cells. On day 13, PD-1 increase more than 20%, CTLA-4 expression ranged from 3 to 6%. Interestingly, PD-L1 was also upregulated (>9%) in both donors, [Figures 1-4]. No significant change in levels of expression of checkpoint molecules in MGMT transduced γδ T cells as compared to untransduced γδ T cells was seen. Moderate downregulation of PD-1 and CTLA-4 on resting (no IL2 and ZOL) γδ T cells when compared to stimulated cultures was also observed [Figure 5].

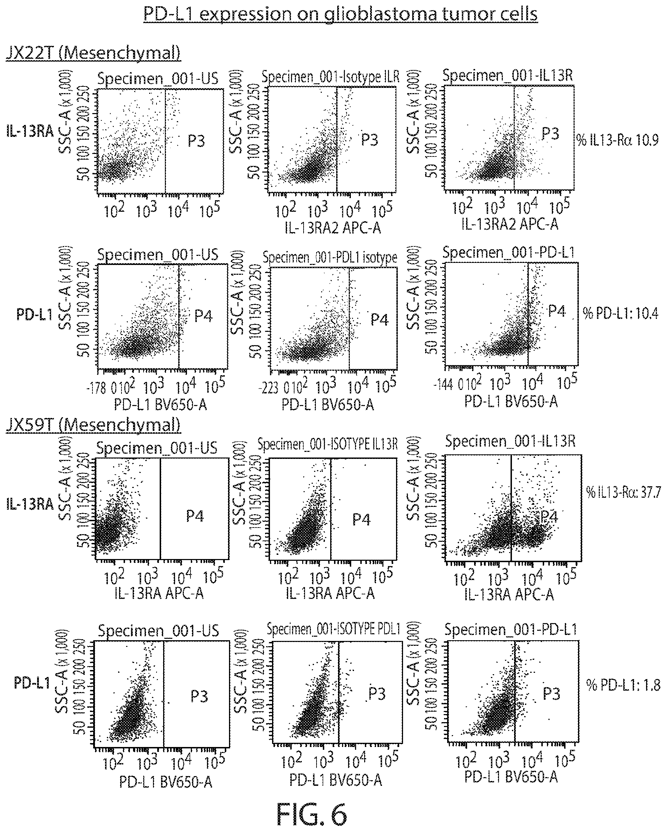

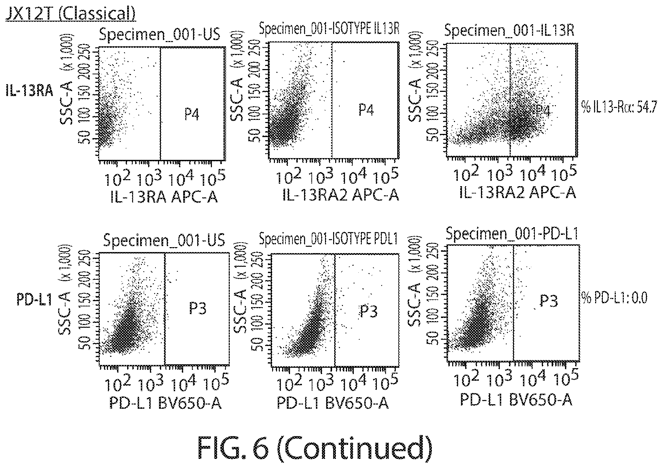

PD-L1 expression on PDX derived Glioma cells was also analyzed by flow cytometry. As compared to isotype controls, PD-L1 receptor expression was low or undetectable. The percentage of PD-L1 expression varied from JX12T (0%), JX22T (1.8%) and JX59T (10.4%) [Figure 6].

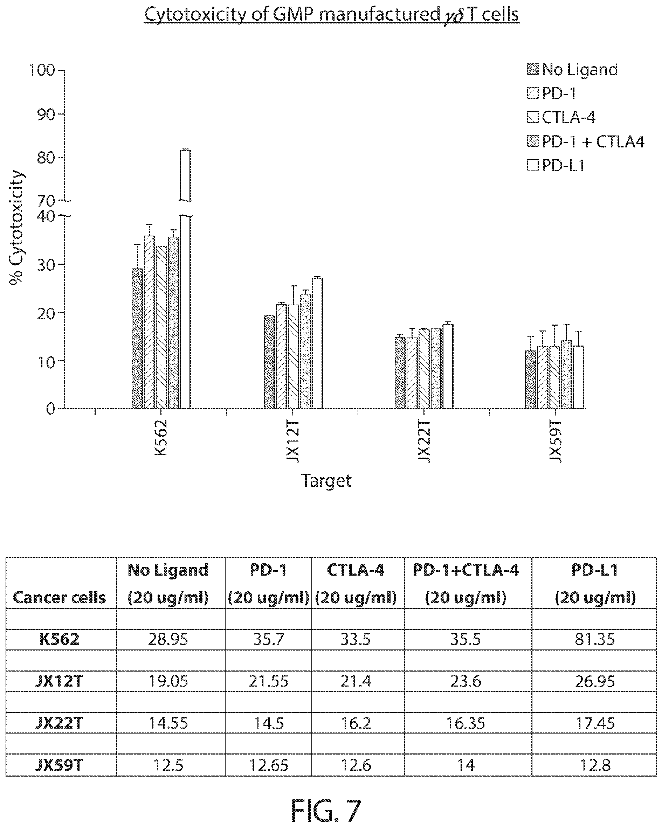

Next, we examined whether the blockade of immune inhibitory receptors had any effect on γδ T cells ability to increase cytotoxicity to cancer cells [Figure 7]. Glioma tumor cells (JX22T, JX12T, JX59T) and K562 (a cell line established from chronic myelogenous leukemia) were challenged with manufactured γδ T cells at 20:1 ratio, in the assay mixture that contained one or more checkpoint antibodies at a concentration of 20 µg/ml. High level of cytolytic activity was observed for K562 [PD-L1 (52%), CTLA-4 (5%), CTLA-4+PD-1 (7%), PD-1 (7). Modest increase in tumor cell lysis was observed for JX12T tumor cells for PD-1 (3%), CTLA-4 (2) PD-1+ CTLA-4 (5) combo and PD-L1 (8) blockade. However, checkpoint blockade did not increase the cytotoxicity of MGMT modified γδ T cells for JX22T AND JX59T.

Our observation that glioblastoma tumor cells does not express significant levels of PD-L1 is in concordance with already published reports, by Garg et al. [Preclinical efficacy of immune-checkpoint monotherapy does not recapitulate corresponding biomarkers-based clinical predictions in glioblastoma. OncoImmunology, 2016]. A study lead by Joseph et al. found high level of PD-L1 expression in tumor infiltrating myeloid cells in the glioblastoma tumors [Immunosuppressive tumor-infiltrating myeloid cells mediate adaptive immune resistance via a PD-1/PD-L1 mechanism in glioblastoma, Neuro-Oncology 9(6), 796-806, 2017]. Since, myeloid/ stromal compartment in the glioma tumor microenvironment express high levels of PD-L1 and our observation that GMP manufactured T cells / *γδ* T cells upregulates PD-1, we propose a comprehensive treatment plan to include systemic treatment using PD-1, PD-L1 blocking antibodies and Temozolomide and localized infusion MGMT expressing γδ T cells to effectively treat glioblastoma and achieve high objective response rate.

## Claims

1. At least one immune checkpoint inhibitor for use in a method for the treatment of cancer in a patient in need thereof, the method comprising the steps of:
i. obtaining an expanded population of isolated cytotoxic immune cells comprising γδ T-cells, wherein greater than about 50% of the γδ T-cells have been genetically modified to express a polypeptide that confers resistance to a chemotherapeutic agent;
ii. administering to the patient an effective amount of the chemotherapeutic agent to which the genetically modified cytotoxic immune cells of step (i) are resistant;
iii. administering to the patient the population of genetically modified cytotoxic immune cells of step (i); and
iv. administering to the patient an effective amount of the at least one immune checkpoint inhibitor wherein the checkpoint inhibitor targets PD-1, PDL1, or CTLA-4.

2. The at least one immune checkpoint inhibitor for use of claim 1, wherein the cancer is selected from glioma, glioblastoma, lymphoma, melanoma, neuroblastoma, non-small cell lung cancer, renal cell carcinoma, and small cell lung cancer.

3. The at least one immune checkpoint inhibitor for use of claims 1 to 2, wherein the polypeptide that confers resistance to the chemotherapeutic agent is O⁶ methylguanine DNA methyltransferase (MGMT), L22Y-DHFR, thymidylate synthase, , or multiple drug resistance-1 protein (MDR1).

4. The at least one immune checkpoint inhibitor for use of any one of claims 1 to 3, wherein the immune checkpoint inhibitor targets PDL1.

5. The at least one immune checkpoint inhibitor for use of any of claims 1 to 3 wherein the checkpoint inhibitor targets PD-1, or CTLA-4.

6. The at least one immune checkpoint inhibitor for use of any preceding claim, wherein the immune checkpoint inhibitor is selected from:
(i) the inhibitor of CTLA-4: tremelimumab;
(ii) the inhibitors of PD-1: AMP224, nivolumab, pembrolizumab and durvalumab; and
(iii) the inhibitors of PD-L1: atelizumab, avelumab, ipilimumab and BMS-936559.

7. The at least one immune checkpoint inhibitor for use of any preceding claim, wherein the chemotherapeutic agent is selected from an alkylating agent; a metabolic antagonist; a DNA demethylating agent; a substituted nucleotide; a substituted nucleoside; an antitumor antibiotic; a plant-derived antitumor agent; cisplatin; carboplatin; etoposide; methotrexate (MTX); trimethotrexate (TMTX); temozolomide; raltitrexed; S-(4-Nitrobenzyl)-6-thioinosine (NBMPR); 6-benzyguanidine (6-BG); a nitrosourea; cytarabine; camptothecin; and a therapeutic derivative of any thereof.

8. The at least one immune checkpoint inhibitor for use of claim 1, wherein the isolated cytotoxic immune cells have been genetically modified to be resistant to two chemotherapeutic agents selected from an alkylating agent; a metabolic antagonist; a DNA demethylating agent; a substituted nucleotide; a substituted nucleoside; an antitumor antibiotic; a plant-derived antitumor agent; cisplatin; carboplatin; etoposide; methotrexate (MTX); trimethotrexate (TMTX); temozolomide; raltitrexed; S-(4-Nitrobenzyl)-6-thioinosine (NBMPR); 6-benzyguanidine (6-BG); a nitrosourea; cytarabine; camptothecin; and a therapeutic derivative of any thereof.

9. The at least one immune checkpoint inhibitor for use of claim 8, wherein the two therapeutic agents are temozolomide and methotrexate.

10. The at least one immune checkpoint inhibitor for use of claim 8, wherein the isolated cytotoxic immune cells express a polypeptide selected from alkyl guanine transferase (AGT) and dihydrofolate reductase.

11. The at least one immune checkpoint inhibitor for use of claim 7, wherein the chemotherapeutic agent is temozolomide and the isolated cytotoxic immune cells express MGMT.

12. The at least one immune checkpoint inhibitor for use of any preceding claim,, wherein administration of the genetically modified immune cells of step (iii) and the administration of the checkpoint inhibitors of step (iv) occurs substantially simultaneously or sequentially.

13. The at least one immune checkpoint inhibitor for use of any preceding claim, wherein the isolated cytotoxic immune cells are derived from human induced pluripotent stem cells (hiPSCs).

14. The at least one immune checkpoint inhibitor for use of claim 1, wherein the population of cytotoxic immune cells comprises about 50% to about 95% γδ T-cells and comprises about 5% to about 25% NK cells.

15. The at least one immune checkpoint inhibitor for use of claim 1, wherein the polypeptide that confers resistance to the chemotherapeutic agent is alkyl guanine transferase (AGT), P140KMGMT, or, dihydrofolate reductase.

## Patentansprüche

1. Mindestens ein Immuncheckpointinhibitor zur Verwendung in einem Verfahren zur Behandlung von Krebs bei einem dessen bedürftigen Patienten, wobei das Verfahren die folgenden Schritte umfasst:
i. das Gewinnen einer expandierten Population isolierter zytotoxischer Immunzellen, die γδ T-Zellen umfassen, wobei mehr als etwa 50 % der γδ T-Zellen so genetisch modifiziert sind, dass sie ein Resistenz gegen ein Chemotherapeutikum verleihendes Polypeptid exprimieren,
ii. die Verabreichung einer wirksamen Menge des Chemotherapeutikums, gegen das die genetisch modifizierten zytotoxischen Immunzellen aus Schritt (i) resistent sind, an den Patienten,
iii. die Verabreichung der Population genetisch modifizierter zytotoxischer Immunzellen aus Schritt (i) an den Patienten und
iv. die Verabreichung einer wirksamen Menge des mindestens einen Immuncheckpointinhibitors an den Patienten, wobei der Immuncheckpointinhibitor auf PD-1, PDL1 oder CTLA-4 zielt.

2. Mindestens ein Immuncheckpointinhibitor zur Verwendung nach Anspruch 1, wobei die Krebserkrankung aus Gliom, Glioblastom, Lymphom, Melanom, Neuroblastom, nichtkleinzelligem Lungenkrebs, Nierenzellkarzinom und kleinzelligem Lungenkrebs ausgewählt ist.

3. Mindestens ein Immuncheckpointinhibitor zur Verwendung nach Anspruch 1 oder 2, wobei es sich bei dem Resistenz gegen ein Chemotherapeutikum verleihenden Polypeptid um O⁶-Methylguanin-DNA-Methyltransferase (MGMT), L22Y-DHFR, Thymidylatsynthase oder Multiple Drug Resistance-1-Protein (MDR1) handelt.

4. Mindestens ein Immuncheckpointinhibitor zur Verwendung nach einem der Ansprüche 1 bis 3, wobei der Immuncheckpointinhibitor auf PDL1 zielt.

5. Mindestens ein Immuncheckpointinhibitor zur Verwendung nach einem der Ansprüche 1 bis 3, wobei der Immuncheckpointinhibitor auf PD-1 oder CTLA-4 zielt.

6. Mindestens ein Immuncheckpointinhibitor zur Verwendung nach einem der vorhergehenden Ansprüche, wobei der Immuncheckpointinhibitor ausgewählt ist aus:
(i) dem CTLA-4-Inhibitor Tremelimumab,
(ii) den PD-1-Inhibitoren AMP224, Nivolumab, Pembrolizumab und Durvalumab und
(iii) den PD-Ll-Inhibitoren Atelizumab, Avelumab, Ipilimumab und BMS-936559.

7. Mindestens ein Immuncheckpointinhibitor zur Verwendung nach einem der vorhergehenden Ansprüche, wobei das Chemotherapeutikum aus einem Alkylierungsmittel, einem Stoffwechselantagonisten, einem DNA-Demethylierungsmittel, einem substituierten Nukleotid, einem substituierten Nukleosid, einem Antitumorantibiotikum, einem pflanzlichen Antitumormittel, Cisplatin, Carboplatin, Etoposid, Methotrexat (MTX), Trimethotrexat (TMTX), Temozolomid, Raltitrexed, S-(4-Nitrobenzyl)-6-thioinosin (NBMPR), 6-Benzylguanidin (6-BG), einem Nitrosoharnstoff, Cytarabin, Camptothecin und einem therapeutischen Derivat eines dieser ausgewählt ist.

8. Mindestens ein Immuncheckpointinhibitor zur Verwendung nach Anspruch 1, wobei die isolierten zytotoxischen Immunzellen so genetisch modifiziert sind, dass sie resistent gegen zwei Chemotherapeutika ausgewählt aus einem Alkylierungsmittel, einem Stoffwechselantagonisten, einem DNA-Demethylierungsmittel, einem substituierten Nukleotid, einem substituierten Nukleosid, einem Antitumorantibiotikum, einem pflanzlichen Antitumormittel, Cisplatin, Carboplatin, Etoposid, Methotrexat (MTX), Trimethotrexat (TMTX), Temozolomid, Raltitrexed, S-(4-Nitrobenzyl)-6-thioinosin (NBMPR), 6-Benzylguanidin (6-BG), einem Nitrosoharnstoff, Cytarabin, Camptothecin und einem therapeutischen Derivat eines dieser sind.

9. Mindestens ein Immuncheckpointinhibitor zur Verwendung nach Anspruch 8, wobei es sich bei den beiden Therapeutika um Temozolomid und Methotrexat handelt.

10. Mindestens ein Immuncheckpointinhibitor zur Verwendung nach Anspruch 8, wobei die isolierten zytotoxischen Immunzellen ein aus Alkylguanintransferase (AGT) und Dihydrofolatreduktase ausgewähltes Polypeptid exprimieren.

11. Mindestens ein Immuncheckpointinhibitor zur Verwendung nach Anspruch 7, wobei es sich bei dem Chemotherapeutikum um Temozolomid handelt und die isolierten zytotoxischen Immunzellen MGMT exprimieren.

12. Mindestens ein Immuncheckpointinhibitor zur Verwendung nach einem der vorhergehenden Ansprüche, wobei die Verabreichung der genetisch modifizierten Immunzellen aus Schritt (iii) und die Verabreichung der Checkpointinhibitoren aus Schritt (iv) im Wesentlichen gleichzeitig oder nacheinander erfolgt.

13. Mindestens ein Immuncheckpointinhibitor zur Verwendung nach einem der vorhergehenden Ansprüche, wobei die isolierten zytotoxischen Immunzellen von humanen induzierten pluripotenten Stammzellen (Human Induced Pluripotent Stem Cells, hiPSCs) stammen.

14. Mindestens ein Immuncheckpointinhibitor zur Verwendung nach Anspruch 1, wobei die Population zytotoxischer Immunzellen etwa 50 % bis etwa 95 % γδ T-Zellen und etwa 5 % bis etwa 25 % NK-Zellen umfasst.

15. Mindestens ein Immuncheckpointinhibitor zur Verwendung nach Anspruch 1, wobei es sich bei dem Resistenz gegen das Chemotherapeutikum verleihende Polypeptid um Alkylguanintransferase (AGT), P140KMGMT oder Dihydrofolatreduktase handelt.

## Revendications

1. Inhibiteur(s) de point de contrôle immunitaire pour une utilisation dans un procédé de traitement d'un cancer chez un patient qui en a besoin, le procédé comprenant les étapes de :
i. obtention d'une population étendue de cellules immunitaires cytotoxiques isolées comprenant des cellules T γδ, plus de 50 % des cellules T γδ ayant été génétiquement modifiées pour exprimer un polypeptide qui confère une résistance à un agent chimiothérapeutique ;
ii. administration au patient d'une quantité efficace de l'agent chimiothérapeutique auquel les cellules immunitaires cytotoxiques génétiquement modifiées de l'étape (i) sont résistantes ;
iii. administration au patient de la population de cellules immunitaires cytotoxiques génétiquement modifiées de l'étape (i) ; et
iv. administration au patient d'une quantité efficace de l'au moins un inhibiteur de point de contrôle immunitaire, l'inhibiteur de point de contrôle ciblant PD-1, PDL1 ou CTLA-4.

2. Inhibiteur(s) de point de contrôle immunitaire pour une utilisation selon la revendication 1, le cancer étant sélectionné parmi un gliome, un glioblastome, un lymphome, un mélanome, un neuroblastome, un cancer du poumon non à petites cellules, un carcinome des cellules rénales et un cancer du poumon à petites cellules.

3. Inhibiteur(s) de point de contrôle immunitaire pour une utilisation selon les revendications 1 à 2, le polypeptide qui confère une résistance à l'agent chimiothérapeutique étant l'O⁶ méthylguanine ADN méthyltransférase (MGMT), L22Y-DHFR, la thymidylate synthase ou la protéine 1 à résistance à plusieurs médicaments (MDR1).

4. Inhibiteur(s) de point de contrôle immunitaire pour une utilisation selon l'une quelconque des revendications 1 à 3, l'inhibiteur de point de contrôle immunitaire ciblant PDL1.

5. Inhibiteur(s) de point de contrôle immunitaire pour une utilisation selon l'une quelconque des revendications 1 à 3, l'inhibiteur de point de contrôle ciblant PD-1 ou CTLA-4.

6. Inhibiteur(s) de point de contrôle immunitaire pour une utilisation selon l'une quelconque des revendications précédentes, l'inhibiteur de point de contrôle immunitaire étant sélectionné parmi :
(i) l'inhibiteur de CTLA-4 : trémélimumab ;
(ii) les inhibiteurs de PD-1 : AMP224, nivolumab, pembrolizumab et duravalumab ; et
(iii) les inhibiteurs de PD-L1 : atélizumab, avelumab, ipilimumab et BMS-936559.

7. Inhibiteur(s) de point de contrôle immunitaire pour une utilisation selon une quelconque revendication précédente, l'agent chimiothérapeutique étant sélectionné parmi un agent alkylant ; un antagoniste métabolique ; un agent de déméthylation d'ADN ; un nucléotide substitué ; un nucléoside substitué ; un antibiotique antitumoral ; un agent antitumoral dérivé de végétaux ; le cisplatine ; le carboplatine ; l'étoposide ; le méthotrexate (MTX) ; le triméthotrexate (TMTX) ; le témozolomide ; le raltitrexed ; la S-(4-nitrobenzyl)-6-thioinosine (NBMPR) ; la 6-benzyguanidine (6-BG) ; une nitrosourée ; la cytarabine ; la camptothecine ; et un dérivé thérapeutique de l'un quelconque de ceux-ci.

8. Inhibiteur(s) de point de contrôle immunitaire pour une utilisation selon la revendication 1, les cellules immunitaires cytotoxiques isolées ayant été génétiquement modifiées pour résister à deux agents chimiothérapeutiques sélectionnés parmi un agent alkylant ; un antagoniste métabolique ; un agent de déméthylation d'ADN ; un nucléotide substitué ; un nucléoside substitué ; un antibiotique antitumoral ; un agent antitumoral dérivé de végétaux ; le cisplatine ; le carboplatine ; l'étoposide ; le méthotrexate (MTX) ; le triméthotrexate (TMTX) ; le témozolomide ; le raltitrexed ; la S-(4-nitrobenzyl)-6-thioinosine (NBMPR) ; la 6-benzyguanidine (6-BG) ; une nitrosourée ; la cytarabine ; la camptothecine ; et un dérivé thérapeutique de l'un quelconque de ceux-ci.

9. Inhibiteur(s) de point de contrôle immunitaire pour une utilisation selon la revendication 8, les deux agents thérapeutiques étant le témozolomide et le méthotrexate.

10. Inhibiteur(s) de point de contrôle immunitaire pour une utilisation selon la revendication 8, les cellules immunitaires cytotoxiques isolées exprimant un polypeptide sélectionné parmi l'alkyl guanine transférase (AGT) et la dihydrofolate réductase.

11. Inhibiteur(s) de point de contrôle immunitaire pour une utilisation selon la revendication 7, l'agent chimiothérapeutique étant le témozolomide et les cellules immunitaires cytotoxiques isolées exprimant MGMT.

12. Inhibiteur(s) de point de contrôle immunitaire pour une utilisation selon une quelconque revendication précédente, l'administration des cellules immunitaires génétiquement modifiées de l'étape (iii) et l'administration des inhibiteurs de point de contrôle de l'étape (iv) ayant lieu sensiblement simultanément ou séquentiellement.

13. Inhibiteur(s) de point de contrôle immunitaire pour une utilisation selon une quelconque revendication précédente, les cellules immunitaires cytotoxiques isolées étant dérivées de cellules souches pluripotentes (hiPSCs) d'origine humaine.

14. Inhibiteur(s) de point de contrôle immunitaire pour une utilisation selon la revendication 1, la population de cellules immunitaires cytotoxiques comprenant d'environ 50 % à environ 95 % de cellules T γδ et comprenant environ 5 % à environ 25 % de cellules NK.

15. Inhibiteur(s) de point de contrôle immunitaire pour une utilisation selon la revendication 1, le polypeptide qui confère une résistance à l'agent chimiothérapeutique étant l'alkyl guanine transférase (AGT), P140KMGMT ou la dihydrofolate réductase.
